# EUROPEAN PATENT SPECIFICATION

(11) **EP 0 934 745 B1**
(45) Date of publication and mention of the grant of the patent: **28.04.2004**
(21) Application number: 99200310.3
(22) Date of filing: 03.02.1999
(51) Int. Cl.: A61K 31/565, A61P 11/06

(54) **Use of androsterone derivatives for inhibiting DNA binding of ap-1 and airway smooth muscle proliferation**
Androsteron-Derivate, deren Verwendung zur Unterdrückung der Bindung des Aktivatorproteins AP-1 an die DNA wie der Vermehrung der glatten Muskelzellen der Atemwege
Dérivés de l'androstérone et leur utilisation pour l'inhibition de la fixation de la protéine activatrice AP-1 à l'ADN et de la prolifération des cellules des muscles lisses des voies respiratoires

(30) Priority: 04.02.1998 US 18782
(43) Date of publication of application: 11.08.1999
(73) Proprietor: CHARLOTTE-MECKLENBURG HOSPITAL doing business as Carolinas Medical Center, Charlotte, NC 28232-2861 (US)
(72) Inventor: Kennedy, Thomas Preston, Richmond, Virginia 23229 (US)
(74) Representative: Lane, Cathal Michael

(56) References cited:
- EP-A- 0 143 764
- WO-A-94/20111
- US-A- 4 529 548
- C.B MAHOOD, ET AL.: "Dehydroepiandrosterone sulphate concentrations in asthmatic patients: pilot study" THE NEW ZEALAND MEDICAL JOURNAL, vol. 97, no. 768, 28 November 1984, pages 805-808, XP002103037

## Description

### BACKGROUND OF THE INVENTION

This invention relates to the use of androsterone derivatives to inhibit proliferation of airway smooth muscle. More specifically, this invention relates to the use of dehydroepiandrosterone (DHEA) and 16-fluorinated and brominated analogs for the relaxation and bronchodilation of airway smooth muscle and the prevention of secretion of proinflammatory cytokines by human airway epithelium.

Previously asthma was defined as episodic reversible airways obstruction. See American Thoracic Society: Medical Section of the National Tuberculosis Association. "Chronic bronchitis, asthma, and pulmonary emphysema. A statement by the committee on diagnostic standards for non-tuberculous disease," *Am*. *Rev*. *Respir*. *Dis*., 85, 762-768 (1962). It is now appreciated that patients with chronic severe asthma can develop irreversible obstruction of airways. For example, see Brown, J.P., Breville, W.H., and Finucane, K.E., "Asthma and irreversible airflow obstruction," *Thorax*, 39, 131-136 (1984); and Juniper, E.F., Kline, P.A., Vanieleghem, M.A., Ramsdale, E.H., O'Byrne, P.M., and Hargreave, F.E., "Effect of long-term treatment with an inhaled corticosteroid (budesonide) on airway hyperresponsiveness and clinical asthma in non-steroid dependent asthmatics," *Am*. *Rev*. *Respir*. *Dis*., 142, 832-836 (1990). This complication develops from architectural remodelling of the airway wall, resulting in increased smooth muscle mass (Bramley, A.M., Thomson, R.J., Roberts, C.R., and Schellenberg, R.R., "Excessive bronchoconstriction in asthma is due to decreased airway elastance," *Eur*. *Respir*. *J*., 7, 337-341 (1994); and Lambert, R.K., Wiggs, B.R., Kuwano, K., Hogg, J.C., and Pare, P.D., "Functional significance of increased airway smooth muscle in asthma and COPD," *J*. *Appl*. *Physiol*., 74, 2771-2781 (1993)) from both hyperplasia and hypertrophy (Ebina, M., Takahasi, T., Chiba, T., and Motomiya, M., "Cellular hypertrophy and hyperplasia of airway smooth muscle underlying bronchial asthma," *Am*. *Rev*. *Respir. Dis*., 148, 720-726 (1993)), as well as remodelling of other elements. Airway smooth muscle thickening, in turn, is thought to contribute to the nonspecific bronchial hyperresponsiveness characteristic of asthma. (James, A.L., Hogg, J.C., Dunn, L.A., and Pare, P.D., "The use of internal perimeter to compare airway size and to calculate smooth muscle shortening," *Am*. *Rev*. *Respir*. *Dis*., 138, 136-139 (1988); and James, A.L., Pare, P.D., and Hogg, J.C., "The mechanics of airway narrowing in asthma," *Am*. *Rev*. *Respir*. *Dis*., 139, 242-246 (1989); and Pare, P.D., Wiggs, B.R., Hogg, J.C., and Bosken, C., "The comparative mechanics and morphology of airways in asthma and in chronic obstructive pulmonary disease," *Am*. *Rev*. *Respir*. *Dis.,* 143, 1189-1193 (1991)). Airway wall remodelling in asthmatics is often clinically refractory to current bronchodilator and anti-inflammatory therapies (See, for example, Brown, *et al*. (1984), supra; and Juniper, *et al*. (1990), *supra*. New treatment strategies are needed that prevent occurrence of this process.

Prior study in vascular tissue has demonstrated that accelerated coronary atherosclerosis in the transplanted heart is reduced by treatment with dehydroepiandrosterone (DHEA). See Eich, D.M., Nestler, J.E., Johnson, D.E., Dworkin, G.H., Ko, D., Wechsler, A.S., and Hess, M.L., "Inhibition of accelerated coronary atherosclerosis with dehydroepiandrosterone in the heterotopic rabbit model of cardiac transplantation," *Circ*., 87, 261-269 (1993).

Dehydroepiandrosterone (DHEA) and its sulfate conjugate (DHEAS) are the major secretory steroidal products of the adrenal gland, but the physiologic role of DHEA remains unknown. See Ebeling, P., and Kovisto, V.A., "Physiologic importance of dehydroepiandrosterone," *Lancet*, 343, 1479-1481 (1994). DHEA and its synthetic analogs are antiproliferative in animal tumor models and malignant cell lines. For example, see Schwartz, A.G., Lewbart, M.L, and Pashko, L.L., "Novel dehydroepiandrosterone analogues with enhanced biological activity and reduced side effects in mice and rats," *Cancer Res.,* 48, 4817-4822 (1988); and Schwartz, A.G, and Pashko, L.L., "Mechanism of cancer preventive action of DHEA. Role of glucose-6-phosphate dehydrogenase," *Ann*. *N*.*Y*. *Acad*. *Sci*., 774, 180-186 (1995). This action has been explained by inhibition of glucose-6-phosphate dehydrogenase, with subsequent blockade of ribonucleoside and deoxyribonucleotide formation (Dworkin, C.R., Gorman, S.D., Pashko, L.L., Cristofalo, W.J., and Schwartz, A.G., "Inhibition of growth of HeLa and WI-38 cells by dehydroepiandrosterone and its reversal by ribo- and deoxyribonucleosides," *Life Sci.*, 38, 1451-1457 (1986); Garcea, R., Daino, L., Frassetto, S., Cozzolino, P., Ruggiu, M.E., Vannini, M.G., Pascale, R., Lenzerini, L., Simile, M. M., Puddu, M., and Feo, F., "Reversal by ribo- and deoxyribonucleosides of dehydroepiandrosterone-induced inhibition of enzyme altered foci in the liver of rats subjected to the initiation-selection process of experimental carcinogenesis," *Carcinogenesis*, 9, 931-938 (1988); Pashko, L.L., Lewbart, M.L., and Schwartz, A.G., "Inhibition of 12-O-tetradecanoylphorbol-13-acetate-promoted skin tumor formation in mice by 16α-fluoro-5-anderosten-17-one and its reversal by deoxyribonucleosides," *Carcinogenesis*, 12, 2189-2192 (1991); Schwartz, *et al*. (1988), *supra*; and Schwartz and Pashko (1995), *supra*)*,* or by interference in mevalonic acid biosynthesis, with reduced protein isoprenylation, impaired localization of *Ras* to the plasma membrane and interruption of *Raf*-kinase-mediated signal transduction cascades. See Schulz, S. and Nyce, J.W., "Inhibition of protein isoprenylation and p21^{ras} membrane association by dehydroepiandrosterone in human colonic adenocarcinoma cells *in vitro*," *Cancer Res*., 51, 653-656 (1991), and Schulz, S., Klann, R.C., Schonfeld, S., and Nyce, J.W., "Mechanisms of cell growth inhibition and cell cycle arrest in human colonic adenocarcinoma cells by dehydroepiandrosterone: Role of isoprenoid biosynthesis," *Cancer Res.,* 52, 1372-1376 (1992).

Reference is also made to US-A-5,660,835 (Nyce) and which disclose the use of dehydroepiandrosterone (DHEA) derivatives to treat asthmatic symptoms, in the later case to mediate most cell alergic reactions. EP 1 033 989 A1 was published after the filing date of the present application.

### SUMMARY OF THE INVENTION

This application describes methods to reduce the growth of airway smooth muscle, whose hyperplasia may lead to fixed airways obstruction and enhanced airways hyperresponsiveness in severe chronic asthma.

This application also describes methods to relax and bronchodilate airway smooth muscle.

This application also describes methods to reduce secretion of proinflammatory cytokines by airway epithelium.

This application further describes a method for the treatment of asthmatic airway remodelling in man. It has been found that the adrenal steroid dehydroepiandrosterone (DHEA) and certain of its analogs reduce growth of immortalized and malignant cell lines. It has also been found that the effects of the subject compounds, dehydroepiandrosterone and its potent analog 16α-bromoepiandrosterone (16α-BrEA) dramatically reduced proliferation in primary cultures of rat tracheal smooth muscle stimulated with fetal bovine serum (FBS) or platelet derived growth factor (PDGF).

The present invention relates to use a compound of the following formula: 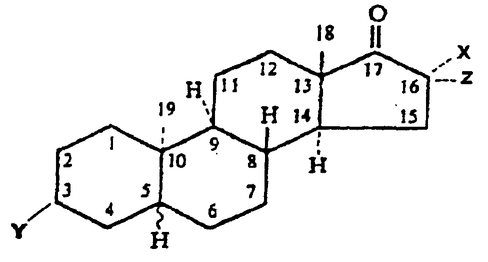 or 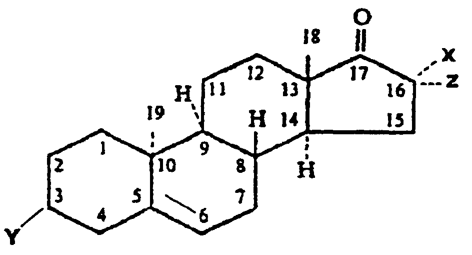 wherein
X is halogen, hydroxy or hydrogen;
Y is hydrogen;
Z is hydrogen,
in the preparation of a medicament comprising a pharmaceutically effective amount of said compound for inhibiting DNA binding of AP-1 and airway smooth muscle proliferation, in the treatment of asthma, in animals.

The preferred steroids for use in this invention include:
16α-bromo-5-androsten-17-one;
16β-bromo-5-androsten-17-one;
16α-fluoro-5-androsten-17-one;
16β-fluoro-5-androsten-17-one;
16α-bromo-5-androstan-17-one;
16β-bromo-5-androstan-17-one;
16α-fluoro-5-androstan-17-one;
16β-fluoro-5-androstan-17-one.

The present invention provides for the treatment of asthma comprising administering to a host, e.g., mammals, a therapeutically effective amount of the subject compounds. As DHEA is less potent than 16α-BrEA or other 16-fluorinated or brominated analogs, the effective dose for DHEA is more.

It has surprisingly been found that growth inhibition is dose-dependent and is not from interference with glucose-6-phosphate dehydrogenase activity or cholesterol metabolism for immortalized or malignant cell lines. Expression of the early response gene *c-fos* remains intact, but DHEA and 16α-BrEA decreased DNA binding of the transcription factor activator protein-1 (AP-1), a later response important for expression of genes mediating DNA synthesis and cell cycle progression.

It has also been found that DHEA and its analogs may impair activation of secondary growth response genes in a fashion analogous to that reported for glucocorticoids, and prove useful for treatment of asthmatic airway remodelling in man.

Furthermore, it has been found that DHEA can relax airway smooth muscle that is contracted by KCl or by the endogenous vagally released bronchoconstrictor mediator acetylcholine, and prove useful as a direct bronchodilator or adjunctive treatment to potentiate beta agonist bronchodilators in the treatment of acute asthma in man.

In addition, it has been found that analogs of DHEA can prevent secretion of proinflammatory cytokines by human airway epithelium, and prove useful to reduce inflammation within the asthmatic airway.

### BRIEF DESCRIPTION OF THE DRAWINGS

Figure 1 shows MTT reduction as an accurate measure of cell numbers counted by hemocytometer;
Figure 2A shows the effect of mitogens on rat airway smooth muscle cell growth (Fetal bovine serum);
Figure 2B shows the effect of mitogens on rat airway smooth muscle cell growth (Platelet derived growth factor);
Figure 3A shows the effect of dehydroepiandrosterone (DHEA) and dehydroepiandrosterone sulfate (DHEAS) on FBS-stimulated growth of airway smooth muscle cells;
Figure 3B shows the effect of dehydroepiandrosterone on airway smooth muscle cell proliferation stimulated by PDGF;
Figure 3C shows the effect of the 16α-bromoepiandrosterone on FBS-stimulated airway smooth muscle proliferation;
Figure 3D shows growth inhibitory effect of 16α-bromoepiandrosterone compared to that of dexamethasone;
Figure 4A shows growth inhibitory effects of dehydroepiandrosterone and 16α-bromoepiandrosterone do not represent measurement artifact;
Figure 4B shows lactate dehydrogenase (LDH) activity in cell supernatants is not increased by 16α-bromoepiandrosterone;
Figure 5A-5D shows that inhibition of airway smooth muscle proliferation by DHEA or 16α-bromoepiandrosterone is not reversed by supplemental ribonucleosides and deoxyribonucleosides added to culture medium;
Figure 6A shows that disruption of cholesterol metabolism does not explain inhibition of airway smooth muscle growth 16α-bromoepiandrosterone;
Figure 6B is an immunoblot that shows that treatment of monolayers with 16α-bromoepiandrosterone does not deplete p21^{ras} from membranes;
Figure 7A is a representative immunoblot of *c-fos* protein from control monolayers treated with FBS and some pretreated with DMSO, 16α-bromoepiandrosterone and A431 cell lysate;
Figure 7B shows the pretreatment for 2 hours with DMSO vehicle or 10 µM 16α-bromoepiandrosterone did not prevent the normal increase of *c-fos* mRNA;
Figure 7C is a summary of experiments shown in Figure 7B;
Figures 8A-8B illustrate that the treatment of airway smooth muscle with dehydroepiandrosterone and 16α-bromoepiandrosterone inhibits DNA binding of the transcription factor AP-1;
Figure 9 illustrates the concentration-effect curve to DHEA and to the vehicle alone (DMSO) in guinea pig main bronchi partially contracted with 3x10⁻² M KCl;
Figure 10 illustrates the concentration-effect curve to DHEA and to the vehicle alone (DMSO) in guinea pig main bronchi partially contracted with 1x10⁻⁵ M acetylcholine (ACh); and
Figure 11 illustrates the concentration-response curve to ACh in the presence and in the absence of 10⁻⁴ M DHEA in guinea pig main bronchi.

### DETAILED DESCRIPTION OF THE INVENTION

The present invention now will be described more fully hereinafter with reference to the accompanying drawings, in which preferred embodiments of the invention are shown. This invention may, however, be embodied in many different forms and should not be construed as limited to the embodiments set forth herein; rather, these embodiments are provided so that this disclosure will be thorough and complete, and will fully convey the scope of the invention to those skilled in the art.

The present invention provides for use a compound of the following formula: 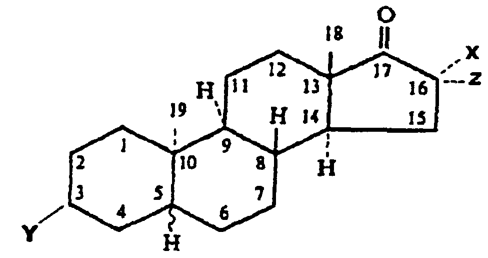 or 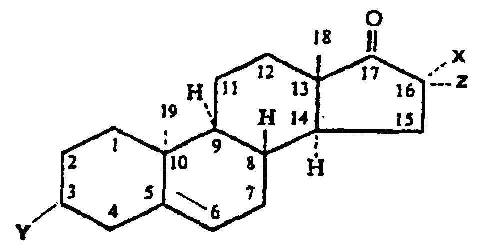 wherein
X is halogen, hydroxy or hydrogen;
Y is hydrogen
Z is hydrogen,
in the preparation of a medicament comprising a pharmaceutically effective amount of said compound, suitable for inhibiting DNA binding of AP-1 and airway smooth muscle proliferation, for example in the treatment of asthma, in an animal, and particularly in man.

Specific illustrative compounds in accordance with the present invention include:
16α-bromo-5-androsten-17-one;
16β-bromo-5-androsten-17-one;
16α-fluoro-5-androsten-17-one;
16β-fluoro-5-androsten-17-one;
16α-bromo-5-androstan-17-one;
16β-bromo-5-androstan-17-one;
16α-fluoro-5-androstan-17-one; and
16β-fluoro-5-androstan-17-one.

As DHEA is less potent than 16-bromo and 16-fluoro analogs the effective dose for DHEA is more. For example, a preferred dosage of DHEA is from 2 to 10 mg/kg/day. A preferred dosage rate for 16-bromo and fluoro substituted analogs described above would be from 0.2 to 2 mg/kg/day.

The medicament can be administered orally and the steroid may be included in a pharmaceutical carrier. Alternately, the medicament can be administered by inhalation in a pharmaceutical carrier such as using conventional metered dose inhaler systems, formulated as a microcrystalline suspension of medicament (micronized to less than 4 microns in size) in a mixture of chlorofluorocarbon propellants such as trichlorofluoromethane and dichlorodifluoromethane with lecithin, so that each inhalation delivers between 25 and 250 µg active drug.

For inhalation, the medicament can also be formulated as a microcrystalline dry powder to be delivered by conventionally available dry powder inhalation systems such as the Spiros dry powder inhalation system, available from Dura Pharmaceuticals, San Diego, CA, or the Inspire inhalation system, available from Inspire, Inc., Palo Alto, CA.

Also, the medicament can be formulated as a component of multilamellar negatively charged liposomes such as by the procedure of Fidler (Fidler, I.J, Raz, A., Fogler, W.E., Kirsh, R., Bugelski, P., and Poste, G., "Design of liposomes to improve delivery of macrophage-augmenting agents to alveolar macrophages", *Cancer Res*. 40:4460-4466, 1980), which involves the use of a mixture of egg phosphatidylcholine and brain phosphatidylserine (Avanti Polar Lipids, Inc., Birmingham, AL) in a molar ratio of 7:3 and formulation of drug-containing liposomes as described by Padmanabhan (Padmanabhan, R.V., Gudapata, R., Liener, I.E., Schwartz, B.A., and Hoidal, J.R., "Protection against pulmonary oxygen toxicity in rats by the intratracheal administration of liposome-encapsulated superoxide dismutase or catalase", *Am*. *Rev*. *Respir*. *Dis*. 132:164-167, 1985). A medicament containing liposomes can be conveniently administered to the respiratory tree by inhalation using conventionally available jet aerosol nebulizer or ultrasonic nebulizer devices, such as the Medicaid Ventstream, Pari-LC Jet, Omron UltraAir, DeVilbiss Aerosonic or Circulair. When administered directly into the lung by inhalation, only one-tenth of the usual oral dose is required, to be administered from once up to three times daily to treat the asthmatic condition.

The steroids used in the present invention may be prepared in accordance with conventional organic syntheses. For example, the steroids used in the present invention can be prepared from steroids which are known or are readily available, such as dehydroepiandrosterone (DHEA), by methods of alkylation, halogenation, hydroxylation or substitution reactions known in the art. Methods for synthesis of a number of the steroids used in the present invention are detailed in: Schwartz, A.G., M.L. Lewbart, and L.L. Pashko, "Novel dehydroepiandrosterone analogues with enhanced biological activity and reduced side effects in mice and rats", *Cancer Res.* 48:4817-4822, 1988.

### EXPERIMENTAL

Studies were conducted to show that the adrenal steroid dehydroepiandrosterone (DHEA) and its analogs reduce growth of immortalized and malignant cell lines.
**Materials**. Male adult Sprague-Dawley rats were purchased from Charles River (Raleigh, NC). Protease inhibitors and guanidine thiocyanate were obtained from Boehringer Mannhein (Indianapolis, IN). Dulbecco's modified Eagle's medium (DMEM), Hanks' balanced salt solution (HBSS), *N*-2-hydroxyethylpiperazine-*N'*-2-ethanesulfonic acid (HEPES), antibiotic-antimycotic (10,000 U penicillin, 10,000 U streptomycin, and 25 µg amphotericin B/ml) and trypsin-ethylenediaminetetraacetic (EDTA) solution were purchased from GIBCO (Grand Island, NY).

Fetal bovine serum (FBS) was purchased from HyClone (Logan, UT). Recombinant human platelet derived growth factor-AA (PDGF-AA) was obtained from R&D Systems, Minneapolis, MN. Dehydroepiandrosterone (DHEA), 16α-bromoepiandrosterone (16α-BrEA), mifepristone (RU486), tris(hydroxymethyl)aminomethane (Tris) and antibody for α-smooth muscle actin were purchased from Sigma Chemical Co. (St. Louis, MO).

Antibodies for p21^{ras} (pan-ras, Ab-3, mouse monoclonal) and *c-fos* (Ab-2, rabbit polyclonal) proteins, horseradish peroxidase-conjugated goat anti-mouse IgG, and A431 cell lysate standard were from Calbiochem (San Diego, CA).

Polyclonal horseradish peroxidase anti-rabbit IgG was from Transduction Laboratories (Lexington, KY). M-MLV reverse transcriptase was from Life Technologies (Gaithersburg, MD). All other materials were obtained from Sigma unless specified.
**Measurement of Cultured Airway Smooth Muscle Proliferation.** Rat tracheal smooth muscle was cultured by euthanizing rats with pentobarbital overdose and removal of their tracheas. The posterior tracheal membrane was isolated, minced and digested twice for 30 minutes at 37°C. in HBSS containing 0.2% type IV collagenase and 0.05% type IV elastase. Each enzyme digest was collected and centrifuged for 5 minutes at 500 g at room temperature. The supernatant was removed and the pellet was resuspended in DMEM supplemented with 10% FBS, nonessential amino acids, penicillin (100 U/ml), streptomycin (100 µg/ml) and amphotericin (250 ng/ml). Cells were seeded in this medium into 25-cm² flasks at 2 x 10⁵ cells per flask and incubated in a humidified atmosphere of 5% CO₂/95% air at 37°C. Upon reaching confluence, cells were detached with 0.25% trypsin-0.002% EDTA solution for passage.

Immunostaining was performed using a polyclonal antibody against α-smooth muscle actin and visualized using an avidin-biotin-immunoperoxidase technique. Smooth muscle cultures demonstrated the typical "hill and valley" appearance under phase-contrast microscopy and stained avidly for α-smooth muscle actin. Preliminary studies demonstrated that culture of cells in the presence of 10% FBS resulted in a linear growth phase up to 120 hours. Cultures from passages 2-9 were used for subsequent experiments.

Proliferation of cultured airway smooth muscle was quantitated using modification of a previously reported colorimetric method based upon metabolic reduction of the soluble yellow tetrazolium dye 3-[4,5-dimethylthiazol]-2yl-2,5-diphenyl tetrazolium bromide (MTT) to its insoluble purple formazan by the action of mitochondrial succinyl dehydrogenase. See Hirst, S.J., Barnes, P.J., and Twort, C.H.C., "Quantifying proliferation of cultured human and rabbit airway smooth muscle in response to serum and platelet derived growth factor," *Am*. *J*. *Respir*. *Cell Mol*. *Biol*., 7, 574-581 (1992). This assay empirically distinguishes between dead and living cells. For proliferation studies, cells were seeded into 24-well uncoated plastic plates at 15,000-50,000 cells per well and cultured with DMEM and mitogens. After 24-96 hours the medium was replaced with 1 ml/well fresh DMEM containing 100 µg/ml MTT and 0.5% FBS, and plates were incubated an additional hour MTT-containing medium was removed, cells were washed twice with 1 ml of sterile Dulbecco's modified phosphate buffered saline without Ca²⁺ or Mg²⁺ (DPBS), 0.5 ml dimethylsulfoxide (DMSO) was added to each well, and the absorbance of the solubilized purple formazan dye was measured at 540 nm on a Shimadzu UV160U spectrophotometer. A total of 4-6 wells was studied at each treatment condition.

Preliminary studies were performed with 50-200 µg/ml MTT incubated for 15 minutes to 3 hours to determine the optimum concentration and incubation time at which the rate of conversion was linear and proportional to the number of cells present. To confirm that 3-[4,5-dimethylthiazol]-2yl-2,5-diphenyl tetrazolium bromide(MTT) reduction was a reliable linear measurement of cell number, freshly detached airway smooth muscle cells were incubated for 1 hour in DMEM containing 10% FBS and 100 µg/ml MTT. Cells were centrifuged 1,000 *g* for 10 min, washed twice with Dulbecco's modified phosphate buffered saline without Ca²⁺ or Mg² (DPBS), extracted with 0.5 ml dimethylsulfoxide (DMSO) and the concentration of formazan was measured as described above.

The optimum concentration of FBS for use as a mitogenic stimulus was determined by incubating 15,000 cells per well with DMEM containing 0.25, 1.0, 5.0 or 10.0% FBS and MTT reduction was determined after 24, 48, 72 or 96 hours. Finally, to confirm that mitogens were stimulating and inhibitors decreasing actual cell numbers, 15,000 cells were incubated with DMEM containing 10% FBS with or without various inhibitors or vehicle. After 24 hours, cells were washed twice with DPBS, fixed and permeabilized by two sequential 5 minute exposures to ice-cold methanol, stained with Giemsa-modified Wright's stain for 3 minutes and washed with DPBS. Cell counts were performed on 10 random fields at 40 power using a 0.01-cm² ocular grid.
**Cell Culture Treatments for Measurement of Airway Smooth Muscle Proliferation.** The effect of dehydroepiandrosterone (DHEA) and 16α-bromoepiandrosterone (16α-BrEA) on cellular proliferation was studied in cultures stimulated with 0.5-10% FBS or 1-50 ng/ml recombinant human platelet derived growth factor (PDGF-AA). In some experiments the glucocorticoid and progesterone antihormone RU486 was added individually or with dehydroepiandrosterone (DHEA) or 16α-bromoepiandrosterone (16α-BrEA) in equimolar quantities in an effort to determine whether inhibitory effects were mediated by DHEA or 16α-BrEA binding to the glucocorticoid protein receptor. See, for example, the method of Le, Y. Y. , and Xu, R.B., "The molecular mechanism of the action of the pharmacological doses of glucocorticoids. Studies on the low-affinity glucocorticoid receptor," *Receptor*, 5, 63-69 (1995). In other studies the growth inhibitory effects of DHEA or 16α-BrEA were compared to those of the glucocorticoids dexamethasone or methylprednisolone.

To determine if DHEA and 16α-bromoepiandrosterone reduced cellular proliferation by inhibition of glucose-6-phosphate dehydrogenase (G6PDH), 80-cm² confluent flasks were incubated with DHEA, 16α-BrEA or 50 µl DMSO vehicle. After 1 hour cells were lysed and G6PDH activity was assayed as described below. In other experiments, G6PDH activity was measured in cell lysates prepared from untreated confluent cultures and treated *in vitro* with DHEA or 16α-BrEA added to the reaction mixture in 5 µl DMSO. To determine if blockade of hexose monophosphate shunt production of ribose sugars was responsible for growth inhibition of airway smooth muscle by DHEA or 16α-BrEA, cells were growth synchronized for 24 hours in 0.5% FBS (15,000 cells/well in 24-well plates), then incubated in DMEM and 10% FBS in the presence or absence of DHEA or 16α-BrEA and 200 µM ribonucleosides (adenosine, guanosine, cytidine, and uridine) or deoxyribonucleosides (deoxyadenosine, deoxyguanosine, deoxycytidine, and thymidine). Ribonucleosides or deoxyribonucleosides were dissolved in 0.5 ml of 1 N HCl and added to DMEM. The pH of the medium was adjusted to 7.1 by titration with 1 N NaOH, and medium was then sterilized by passage through a 0.2 µM filter. Growth was measured by MTT reduction after 24, 48 and 96 hours.

To determine if 16α-bromoepiandrosterone reduced cellular proliferation by interfering with mevalonic acid synthesis, cells were stimulated with FBS and grown in the presence or absence of 16α-BrEA, with or without addition of 6 mM DL-mevalonic acid lactone to growth medium. After 36 hours growth was measured by MTT reduction. To evaluate whether isoprenylation and membrane localization of *Ras* was impaired, confluent cells on 75-cm² Petri dishes were growth arrested for 24 hours in 0.5% FBS and DMEM with and without 16α-BrEA or DMSO vehicle. Some dishes were then stimulated 30 minutes with 10% FBS in DMEM. Cells were lysed, membranes were isolated and immunoblotting was performed as described below to determine if treatment with DHEA and 16α-BrEA depleted p21^{ras}.

To determine if 16α-BrEA impaired expression of early response genes important for cellular proliferation, confluent 75-cm² Petri dishes of airway smooth muscle cells were growth arrested by incubation for 24 hours in DMEM with 0.5% FBS. Monolayers were pretreated 2 hours with 10 µM 16α-BrEA or DMSO vehicle, and stimulated by exposure to DMEM with 10% FBS for 30 minutes. Cells were then lysed and *c-fos* mRNA was measured by the reverse transcriptase-polymerase chain reaction, as described below. To study whether levels of *c-fos* protein were affected, confluent monolayers on 6-well plates were growth arrested for 24 hours in DMEM with 0.5% FBS. Cells were then pretreated 2 hours with 16α-BrEA or DMSO vehicle and stimulated with 10% FBS in DMEM for 15, 30 and 60 minutes. Cells were then lysed and *c-fos* protein was assayed by immunoblotting as described below.

To study the effect of DHEA and 16α-BrEA on activation of AP-1, a secondary response important in cellular growth and proliferation (Angel and Karin, (1991), supra) confluent monolayers of airway smooth muscle in 75-cm² Petri dishes were growth arrested for 24 hours in 0.5% FBS and DMEM and pretreated 2 hours with DHEA, 16α-BrEA or DMSO vehicle. Cells were then stimulated with 10% FBS in DMEM for 6 hours, nuclear protein was harvested, and electrophoretic mobility shift assays were performed as described below to determine if treatment with DHEA and 16α-BrEA impaired DNA binding of AP-1.
**Measurement of Cytotoxicity and Apoptosis.** To assess for cytotoxicity, DHEA or 16α-BrEA were added to wells of airway smooth muscle cells previously grown to confluence in DMEM and 10% FBS. After 24 hours, media was microfuged 5 minutes and supernatant was assayed for lactate dehydrogenase activity using a commercially available assay (DG-1340K from Sigma). Cells were washed twice in DPBS and exposed to trypan blue dye (0.04% in Hanks' balanced salt solution). Cell counts were performed in five random fields using a 0.01-cm² ocular grid to quantitate the average number of damaged or dead cells that accumulated dye.

To determine if DHEA or its analogs induced programmed cell death, confluent cultures in 6 well plastic plates were treated with 50 or 250 µM DHEA, 10 or 50 µM 16α-BrEA, or vehicle (25 µl DMSO). After 2 hours cells were washed twice with DPBS and scraped on ice into 1.5 ml microfuge tubes and centrifuged 250 g for 5 minutes at 4°C. The cell pellet was gently resuspended in 30 µl DPBS and lysed by addition of 30 µl lysis buffer (80 mM EDTA, 1.6% [wt/vol] sodium lauryl sarcosinate and 5 mg/ml proteinase K in 200 mM Tris-HCl buffer, pH 8.0). Lysate was incubated at 50°C for 1.5 hours. RNAse A (0.2 mg/ml) was added and the lysate was incubated for another 30 minutes at 37°C. DNA bands were separated along with a DNA ladder standard on a 1% agarose gel at 60 V for 1 hour, intercalated with ethidium bromide, and visualized and photographed under ultraviolet light.
**Measurement of Glucose-6-Phosphate Dehydrogenase Activity.** Cultures were washed three times with cold DPBS on ice, scraped into ice cold buffer (10 mM MgCl₂ in 50 mM Tris, pH 8.0) and sonicated on ice. G6PDH activity was then assayed by the method of Jones and Andrews (Jones, J.T., and Andrews, S.G., "Glucose-6-phosphate dehydrogenase activity in somatic and germinal cells of the mouse testis," *J*. *Reproduc*. *Fertility*, 54, 357-362 (1978)) in a reaction mixture containing 50-200 µl cell lysate, 20 µl of 10 mM β-nicotinamide adenine dinucleotide phosphate (NADP) and 20 µl of 10 mM D-glucose-6-phosphate (G6P) in a total volume of 1 ml buffer (10 mM MgCl₂ in 50 mM Tris, pH 8.0). The reaction was initiated by addition of G6P. The linear increase in absorbance at 340 nm was monitored at 25°C for 300 seconds. Activity was expressed as units/mg protein, where 1.0 unit will oxidase 1.0 µmole of G6P to 6-phospho-D-gluconate per min in the presence of NADP. Protein was measured using the BCA protein assay (Rockford, IL).
**Immunoblot Assay for p21**^{**ras**} **and *c-fos* Proteins**. For measurement of p21^{ras}, cells were washed twice-with cold DPBS and swelled on ice for 15 minutes with 500 µl cold lysis buffer (10 mM HEPES, 1 mM MgCl₂, 1 mM EDTA, 1 µM pepstatin, 2 µg/ml aprotinin, and 1 mM phenylmethylsulfonyl fluoride). See Schulz and Nyce (1991), *supra*. Cells were then scraped into 1.5 ml polypropylene tubes, homogenized and clarified by centrifugation at 3,000 g for 1 minute at 4°C. The postnuclear supernatant was centrifuged 100,000 g for 30 minutes at 4°C. Supernatants were collected and pelleted membranes were resuspended in detergent buffer (1% Triton X-100, 1% sodium deoxycholate, 0.1% SDS, 150 mM NaCl, 1 µM pepstatin, 2 µg/ml aprotinin and 1 mM phenylmethylsulfonyl fluoride in 25 mM Tris, pH 7.4). Aliquots of supernatant and membrane fractions were removed for protein determination using the BCA protein assay. Bromophenol blue and β-mercaptoethanol were added to the remaining sample to a final concentration of 0.002% (wt/vol) and 5% (vol/vol), respectively. Lysates were then boiled for 5 minutes at 100°C and stored at -80°C until immunoblotting was performed.

For measurement of *c-fos* protein, monolayers were placed on ice, washed twice with cold DPBS, scraped into 0.5 ml boiling buffer (10% [vol/vol] glycerol and 2% [wt/vol] sodium dodecyl sulfate [SDS] in 83 mM Tris, pH 6.8) and sonicated. Aliquots were removed for protein determination, bromophenol blue and β-mercaptoethanol were added and lysates were boiled and stored as outlined above.

Proteins in defrosted samples were separated by SDS-polyacrylamide gel electrophoresis on 10% polyacrylamide gels for *c-fos* and 15% gels for p21^{ras} (15 µg protein/lane) with the use of O'Farrell-Laemmli buffer (0.025 M Tris, 0.192 M glycine, and 0.1% [wt/vol] SDS; pH 8.3). See method of Buckley, B.J., and Whorton, A.R., "Ca²⁺-independent arachidonic acid release by vascular endothelium requires protein synthesis de novo," *Biochem*. *J*., 300, 445-449 (1994). Duplicate gels were stained with Coomassie blue (Coomassie blue stain R-250 from Sigma in 40% methanol and 7% acetic acid) to evaluate the relative amount of protein loaded into each lane. Proteins were transferred to nitrocellulose by the wet transblot method in transfer buffer (0.025 M Tris, 0.192 M glycine, 2.6 mM SDS, and 20% [vol/vol] methanol; pH 8.8) at 200 mAmp for 3 hours. Blots were blocked for 1 hour at room temperature in 10 mM Tris (pH 7.5) containing 100 mM NaCl, 0.01% (vol/vol) Tween 20, and 5% nonfat dry milk. Blots were incubated for 1 hour at room temperature with 2.5 µg/ml of either pan-ras or *c-fos* antibodies in blocking buffer. After rinsing 5 times for 5 minutes each in rinse buffer (10 mM Tris [pH 7.5] containing 100 mM NaCl and 0.01% [vol/vol] Tween 20), blots were incubated for 1 hour at room temperature with the enzyme conjugate anti-mouse immunoglobulin G (IgG)-horseradish peroxidase (HRP; pan-ras MAb) or anti-rabbit IgG/HRP (*c-fos* PAb) diluted 1:2,000 in blocking buffer as the secondary antibody. Immunoblots were rinsed again 5 times for 5 minutes each in rinse buffer and immunodetected via an enhanced chemiluminescence method (ECL Western blotting detection system, Amersham Life Science, Buckinghamshire, England). Estimations of molecular weight were based on comparisons to prestained molecular weight markers (Bio-Rad Laboratories, Hercules, CA) transferred to nitrocellulose. Autoradicgraphic film (X-OMAT AR, Eastman Kodak, Rochester, NY) was exposed for 10, 15, or 60 seconds to immunoblots.
**Reverse Transcriptase-Polymerase Chain Reaction (RT-PCR)**. Monolayers were washed twice with DPBS, and cells were lysed with 4 M guanidine thiocyanate, 50 mM sodium citrate, 0.05% Sarkosyl, and 0.01 M dithiothreitol. After scraping, lysates were-sheared with four passes through a 22 gauge needle. RNA was pelleted by ultracentrifugation through 5.7 M cesium chloride and 0.1 M EDTA. See method of Becker, S., Koren, H.S., and Henke, D.C., "Interleukin-8 expression in normal nasal epithelium and its modulation by infection with respiratory syncytial virus and cytokines tumor necrosis factor, interleukin-1, and interleukin-6," *Am*. *J*. *Respir*. *Cell Mol*. *Biol*., 820-27 (1993).

RNA (100 ng) was reverse-transcribed using M-MLV reverse transcriptase. The resultant cDNA was PCR-amplified for 29 and 36 cycles for β-actin and *c-fos*, respectively, using rat gene specific sense and antisense primers based on sequences determined from GenBank and published in: Dashtaki, R., Whorton, A.R., Murphy, T.M., Reed, W., and Kennedy, T.P., "Dehydroepiandrostserone and analogs inhibit DNA binding of AP-1 and airway smooth muscle proliferation", *J*. *Pharmacol*. *Exper*. *Ther*. 1998, in press. PCR-amplified DNA was separated on 2% denaturing agarose gel, intercalated with ethidium bromide, and visualized and photographed under ultraviolet light. The resulting polaroid negative was quantitated using a Bio Image Analyzer (Bio Image, Ann Arbor, MI). The intensity of the β-actin cDNA bands (a housekeeping gene unaffected by stimulation with FBS) for each sample was then used to normalize differences between samples.
**Electrophoretic Mobility Shift Assay (EMSA).** Monolayers were washed twice in cold DPBS and equilibrated 10 minutes on ice with 0.7 ml cold cytoplasmic extraction buffer (CEB) (10 mM Tris, pH 7.9, 60 mM KCl, 1 mM EDTA, 1 mM dithiothreitol) with protease inhibitors (PI) (1 mM Pefabloc, 50 µg/ml antipain, 1 µg/ml leupeptin, 1 µg/ml pepstatin, 40 µg/ml bestatin, 3 µg/ml E-64 and 100 µg/ml chymostatin). The detergent Nonidet P-40 (NP-40) was added to a final concentration of 0.1% and cells were dislodged with a cell scraper. Nuclei were pelleted by centrifugation and washed with CEB/PI. Nuclei were then incubated for 10 minutes on ice in nuclear extraction buffer (NEB) (20 mM Tris, pH 8.0, 400 mM NaCl, 1.5 mM MgCl₂, 1.5 mM EDTA, 1 mM dithiothreitol and 25% glycerol) with PI, spun briefly to clear debris and stored at -80°C until performance of electrophoretic mobility shift assays. EMSAs employed wild type (5'-TTCCGGCTGACTCATCAAGCG 3' and 3' AAGGCCGACTGAGTAGTTCGC 5') consensus sequences for AP-1 (Lee, *et al*. (1987), *supra*), end-labeled by phosphorylation with [γ³²P]-ATP and T4 polynucleotide kinase.

DNA-protein binding reactions were performed with 2 µg of nuclear protein (as determined by the Bradford dye binding mechod) and 0.3 ng of ³²P-end-labelled double-stranded DNA probe incubated 10 minutes at room temperature in 10 mM Tris, pH 7.9, 50 mM NaCl, 2.5 mM EDTA, 1 mM dithiothreitol, 5 µg bovine serum albumin, 0.1 µg poly dI-dC, and 4% Ficoll. Competition experiments were performed with 10X unlabeled wild-type oligonucleotide sequences. Samples were electrophoresed on a 5% nondenaturing polyacrylamide gel in Tris-glycine-EDTA (TGE, 120 mM glycine and 1 mM EDTA in 25 mM Tris, pH 8.5) buffer. Gels were dried and analyzed by exposure to a phosphorimaging screen (Molecular Dynamics, Sunnyvale, CA).
**Effect on bronchial smooth muscle contraction in vitro.** To study the effect of DHEA on bronchial smooth muscle in vitro, male Hartley guinea-pigs (Charles River Laboratories, Inc., Wilmington, MA) were anaesthetized with sodium pentobarbital (pentobarbital sodium (Abbott Laboratories) (200 mg/kg intraperitoneally). Then trachea and lungs were quickly removed and immersed in oxygenated Krebs-Henseleit solution (K-H) containing (mM) : 115 NaCl, 25 NaHCO₃, 1.38 NaH₂PO₄, 2.5 KCl, 2.46 MgSO₄, 1.9 CaCl₂, and 5.56 dextrose, aerated with 95% O₂ and 5% CO₂.

The main bronchi were dissected free of loose connective tissue, under a stereo-microscope (Olympus SZH10), and bronchial rings were obtained and mounted in double jacketed organ baths filled with K-H at 37°C and continuously aerated with a 95% O₂ and 5% CO₂ gas mixture, which produced a pH of 7.4. The bronchial rings were connected to force displacement transducers (Grass FTO3) for continuous recording of isometric tension on paper polygraph (Gould RS3800) and allowed to equilibrate for 90 minutes. After the equilibration period 1mM acetylcholine was administered and the response monitored. Preparations which did not respond to acetylcholine were rejected, the bronchial rings rinsed with fresh K-H and the experiments continued.

Bronchial rings were partially contracted with 3·10⁻² M KCl or 10⁻⁵ M acetylcholine (ACh) , then the effect of DHEA was studies by increasing cumulatively its concentration at log intervals from 10⁻³ to 10⁻⁴ M and measuring the changes produced in active tension.

A concentration of 3 x 10⁻² M KCl was chosen on the basis of preliminary experiments, in which from concentration-response curves to KCl (10⁻² - 10⁻¹ M) it was determined that the concentration of KCl eliciting 50% of the maximum response (KCl-EC₅₀) in the experimental set up. Maximum response to KCl was obtained at a concentration of 6.2 x 10⁻² M (geometric mean, GSEM=1.21), while KCl-EC₅₀ was 2.8 x 10⁻² M (geometric means, GSEM =1.28). Similarly, 10⁻⁵ M acetylcholine (ACh) was chosen as the concentration of ACh eliciting 50% of the maximum response.

The administration of 3 x 10⁻² M KCl produced a contractile response that declined slightly after four hours, i.e., (the time needed to complete the concentration-effect curve to DHEA) 22.12±6.07% (n=3) lower than the initial active force. No decline of the contractile response was observed after administration of 10⁻⁵ M acetylcholine.

Since DHEA was dissolved in dimethyl sulfoxide (DMSO), four additional rings were contracted with 3 x 10⁻² M KCl (n=5) or 10⁻⁵ M ACh (n=5) to determine whether this vehicle produced any alteration of the active force. The same amount of DMSO used in DHEA experiments was administered, and it was found that after four hours the active tension produced in response to 3 x 10⁻² KCL was reduced by 31.13±7.21%, which is mainly accounted for by the time effect described above. No effect of DMSO was observed on the active tension produced in response to 10⁻⁵ M ACh. The maximum final concentration of DMSO in the organ bath was 0.15%.

Three of the strips used to study DHEA were rinsed with fresh K-H at the end of the experiment, treated with 1 mM acetylcholine, and the response monitored. The results showed the smooth muscle functionality was not altered by DHEA.

In a second set of experiments either a single dose of 10⁻⁵ M ACh was administered or a concentration-response curve to ACh was performed in the presence or absence of 10⁻⁴ M DHEA (administered 15 minutes before ACh). The single dose of ACh was used to measure the rate of tension development, while the concentration-response study was used to evaluate whether DHEA affected sensitivity to ACh.
**Effect on cytokine secretion by human airway epithelium in vitro.** The effect of 16α-BrEA on cytokine secretion by cultured human epithelium was studied in BEAS-2B cells, BEAS-23 cells, an SV-40 immortalized human respiratory epithelial cell line previously studied as a model of how the human airway epithelium secretes pro-inflammatory cytokines in response to viruses and air pollutants (Subasuste, M.C., Jacoby, D.B., Richards, S.M, and Proud, D., "Infection of a human respiratory epithelial cell line with rhinovirus. Induction of cytokine release and modulation of susceptibility to infection by cytokine exposure", *J*. *Clin*. *Invest*. 967:549-557, 1995; Noah, T.L., and Becker, S., "Respiratory syncytial virus-induced cytokine production by a human bronchial epithelial cell line", *Am*. *J*. *Physiol*. 265:L472-L478, 1993; Devlin, R.B., McKinnon, K.P., Noah, T, Becker, S., and Koren, H.S., "Ozone-induced release of cytokines and fibronectin by alveolar macrophages and airway epithelial cells", *Am*. *J*. *Physiol*. 266 (*Lung Cell*. *Mol*. *Physiol*. 10):L612-L619, 1994) BEAS-2B cells were grown to confluence in 12 well plascic plates and pretreated for 1 hr with 16α-bromoepiandrosterone (BrEA) in the concentrations noted or dimethylsulfoxide vehicle (5 µl). Cells were then stimulated with tumor necrosis factor alpha (TNFα, 40 ng/ml) or Residual Oil Fly Ash (ROFA, 50 µg/ml), an air pollution particulate known to stimulate cytokine secretion by inhibiting membrane phosphatases (Samet, J.M, Stonehuerner, J., Reed, W., Devlin, R.B., Dailey, L.A., Kennedy, T.P., Bromberg, P.A., and Ghio, A.J., "Disruption of protein tyrosine phosphate homeostasis in human bronchial epithelial cells exposed to residual oil fly ash", *Am*. *J*. *Physiol*. 272 (*Lung Cell*. *Mol*. *Physiol*. 16):L426-L432, 1997). After 24 hr (for TNF stimulated cells) or 6 hr (for ROFA stimulated cells) media was collected and cencrifuged to remove debris. The concentration of interleukin-8 (IL-8) was assayed by commercial ELISA (R & D Systems).
**Statistical Analysis.** Data are expressed as mean values ± standard error (SEM), except when differently specified. The minimum number of replicates for measurements in studies of airways smooth muscle proliferation was four, unless otherwise indicated. Replicates of 4-6 experiments per treatment group were studied, except where indicated. Differences between multiple groups were compared using one-way analysis of variance for repeated measures. The post-hoc test used was the Newman-Keuls multiple comparison test. Differences between two groups was measureds using the two-tailed Student's t test. Significance was assumed at p < 0.05.

**TABLE I**

| **PERCENT INHIBITION OF GLUCOSE 6-PHOSPHATE DEHYDROGENASE** **ACTIVITY BY DHEA AND 16α-BrEA** | | | | | | |
|---|---|---|---|---|---|---|
| | BrEA (µM) | | DHEA (µM) | | | |
| Assay Conditions | 25 | 50 | 25 | 50 | 100 | 250 |
| | | | | | | |
| Human Erythrocyte G6PDH | 47±6 | 57±9 | - | - | - | - |
| Airway Smooth Muscle Lysate | 49±0 | 71±8 | 77±3 | 82±3 | 93±3 | - |
| Intact Airway Smooth Muscle | - | - | 0 | 0 | 6±7 | 24±9 |

Activity is expressed as % inhibition of DMSO vehicle-treated controls by 16α-bromoepiandrosterone (BrEA) or dehydroepiandrosterone (DHEA). DMSO alone caused no reduction in G6PDH activity under assay conditions. Each bar represents the mean of at least 3 experiments. Methods are described in the text. Assays of human erythrocyte glucose 6-phosphate dehydrogenase (G6PDH) were performed with 0.01 U/ml of Type XXVI G6PDH from human erythrocytes (Sigma), with inhibitors added. Assays of G6PDH in airway smooth muscle cell lysates were performed on 100 µl of cell lysate, to which inhibitors were added. Intact airway smooth muscle monolayers were pretreated with inhibitors 1 hour before harvest of cells and assay of G6PDH using 100 µl of cell lysate. All assays were performed in triplicate.

**TABLE II**

| **EFFECT OF 16α-BROMOEPIANDROSTERONE ON** **INTERLEUKIN-8 SECRETION** **BY CULTURED HUMAN RESPIRATORY EPITHELIUM** | |
|---|---|
| | IL-8 (pg/ml) |
| TNF Stimulated BEAS Cells | |
| Control Media | 34 ± 4 |
| TNF | 141 ± 29 |
| TNF + DMSO | 93 ± 14 |
| TNF + BrEA 2µM | 104 ± 31 |
| TNF + BrEA 10 µM | 52 ± 18 |
| TNF + BrEA 50 µM | 27 ± 2 |

| ROFA Stimulated BEAS Cells | |
|---|---|
| Control Media | 36 ± 1 |
| ROFA | 889 ± 150 |
| ROFA + DMSO | 410 ± 18 |
| ROFA + BrEA 25 µM | 123 ± 5 |

BEAS-2B cells, an SV-40 immortalized human respiratory epithelial cell line were grown to confluence in 12 well plastic plates and pretreated for 1 hr with 16α-bromoepiandrosterone (BrEA) in the concentrations noted or dimethylsulfoxide vehicle (5 µl). Cells were then stimulated with tumor necrosis factor alpha (TNFα, 40 ng/ml) or Residual Oil Fly Ash (ROFA, 50 µg/ml), an air pollution particulate known to stimulate cytokine secretion by inhibiting membrane phosphatases. After 24 hours (for TNF stimulated cells) or 6 hours (for ROFA stimulated cells) media was collected and centrifuged to remove debris. The concentration of interleukin-8 (IL-8) was assayed by commercial ELISA (R & D Systems). Results are means ± standard errors of 4-6 experiments per treatment group. DMSO vehicle, as an antioxidant, reduces IL-8 secretion (a known effect) but BrEA inhibits much more profoundly.

### RESULTS

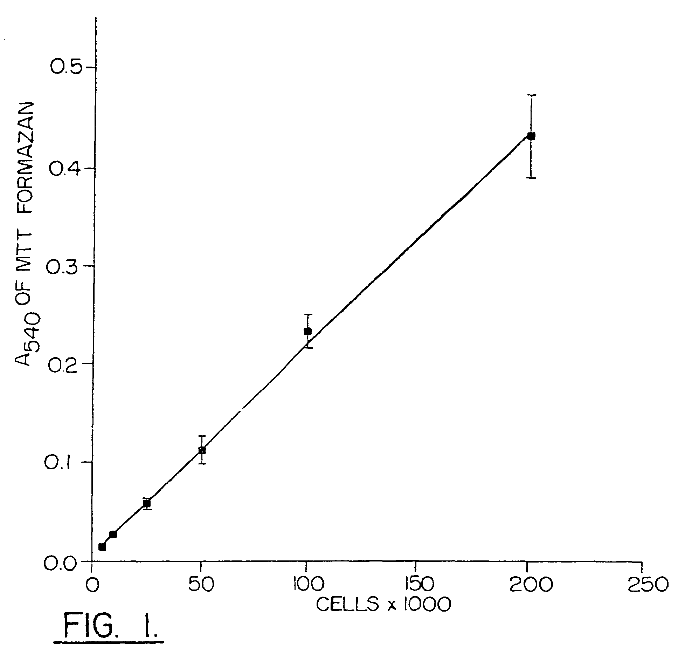

**Effect on Airway Smooth Muscle Proliferation.** As shown in Figure 1, estimation of cell numbers by MTT reduction correlates closely with actual cell numbers counted using a hemocytometer. The proliferation of cultured airway smooth muscle was quantitated using metabolic reduction of the soluble yellow tetrazolium dye 3-[4,5-dimethylthiazol]-2yl-2,5-diphenyl tetrazolium bromide (MTT) to its insoluble purple formazan by the action of mitochondrial succinyl dehydrogenase. See Hirst, *et al*. (1992), *supra*. Freshly detached airway smooth muscle cells were incubated for 1 hour in DMEM containing 100 µg/ml MTT and 10% FBS. Cells were centrifuged 1,000 g for 10 minutes, washed twice with sterile Dulbecco's modified phosphate buffered saline without Ca²⁺ or Mg²⁺ (DPBS), extracted with 0.5 ml dimethylsulfoxide (DMSO) and the absorbance of the solubilized purple formazan dye was measured at 540 nm. A total of 6 experiments was performed at each cell concentration. The absorbance of the MTT formazan reduction product (A₅₄₀) correlated against cell numbers counted by hemocytometer with an R² = 0.9851.

For proliferation studies, cells were seeded into 24-well uncoated plastic plates and cultured with DMEM and mitogens. After 24-96 hours the medium was replaced with 1 ml/well fresh DMEM containing 100 µg/ml MTT and 0.5% FBS, and plates were incubated an additional hour. MTT-containing medium was removed, cells were washed twice with 1 ml of DPBS, 0.5 ml DMSO was added to each well, and cell number was determined by the absorbance of the solubilized purple formazan measured at 540 nm. FBS promoted airway smooth muscle cell growth in a dose-dependent manner, with the greatest stimulus provided by 10% FBS.

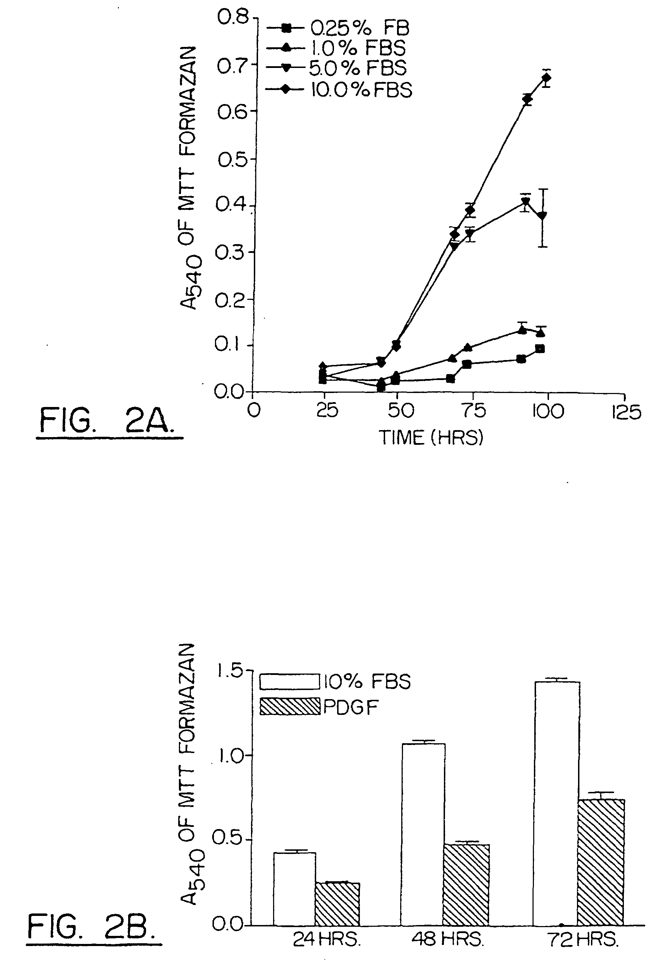

The results in Figure 2A show that fetal bovine serum (FBS) promoted dose dependent growth from 24 to 96 hours. Each observation represented the mean of 6 experiments with 15,000 cells per well.

As shown in Figure 2B recombinant human platelet derived growth factor-AA (PDGF at 50 ng/ml) also stimulated cell growth, but was not as potent as FBS. Each observation represents the mean of 6 experiments with 50,000 cells per well.

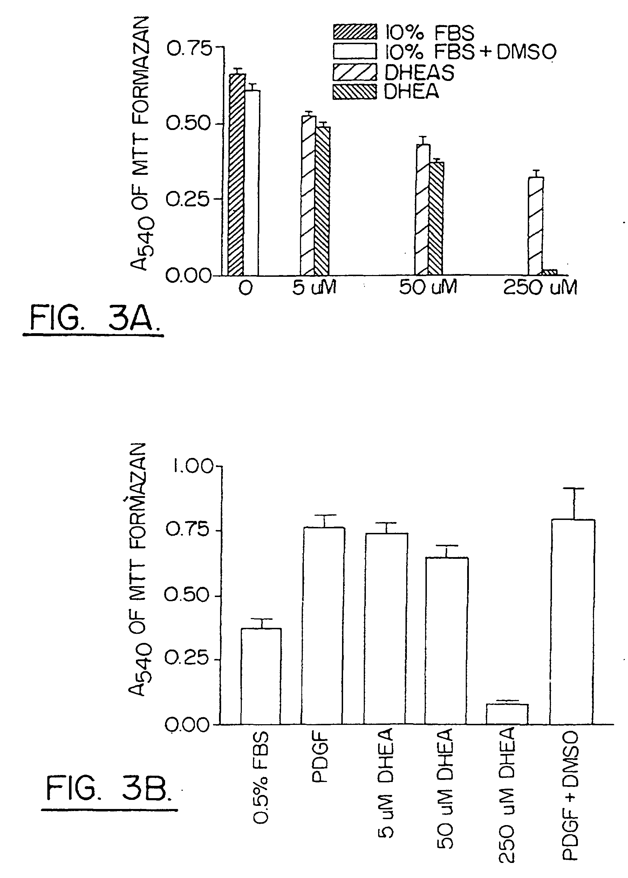

In the next experiment DHEA and its analogs inhibited mitogen-stimulated airway smooth muscle proliferation. Cells were cultured and cell numbers were quantitated as described above and in Figure 2. Except where indicated, cells were cultured with either 10% FBS or 50 ng/ml PDGF, 10% FBS or 50 ng/ml PDGF + 5 µl DMSO vehicle, and 10% FBS or 50 ng/ml PDGF + inhibitors in DMSO added to each well. Figure 3A shows the effect of DHEA and DHEA-sulfate on growth stimulated by 10% FBS after culture for 92 hours. Each bar represents the mean MTT formazan absorbance in 6-12 experiments produced by 15,000 cells/well cultured for 92 hours. Similar results were noted in experiments at 72 hours. DHEA was more active than DHEA-sulfate at high concentrations.

As shown in Figure 3B, DHEA also inhibites cell proliferation stimulated by PDGF. Each bar represents the mean of 4 experiments with 50,000 cells/well cultured for 72 hours.

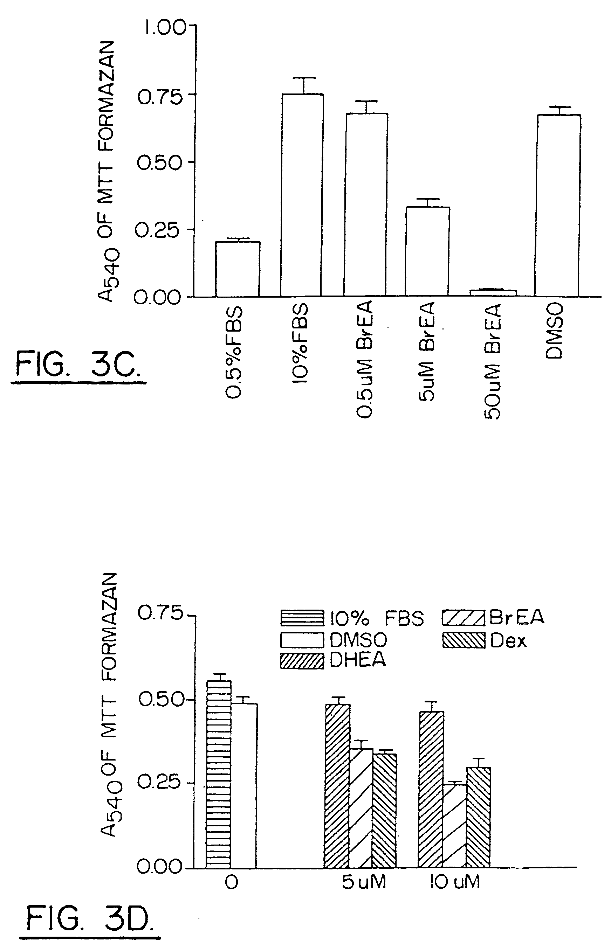

As shown in Figure 3C, 16α-bromoepiandrosterone (16α-BrEA) was an even more potent inhibitor of FBS-induced cell proliferation than DHEA. Each bar represents the mean of 4 experiments with 15,000 cells/well cultured for 96 hours. A negative control incubated with 0.5% FBS is also shown for comparison. Similar results were noted in experiments at 48 hours.

As shown in Figure 3D, 16α-BrEA is also more potent than the glucocorticoid dexamethasone (50% inhibitory concentration = 7.5 µM for 16α-BrEA vs 10 µM for dexamethasone), especially at higher concentrations. Each bar represents the mean of 6-18 experiments with 50,000 cells/well cultured in 10% FBS for 48 hours. Similar results were observed in experiments performed with methylprednisolone.

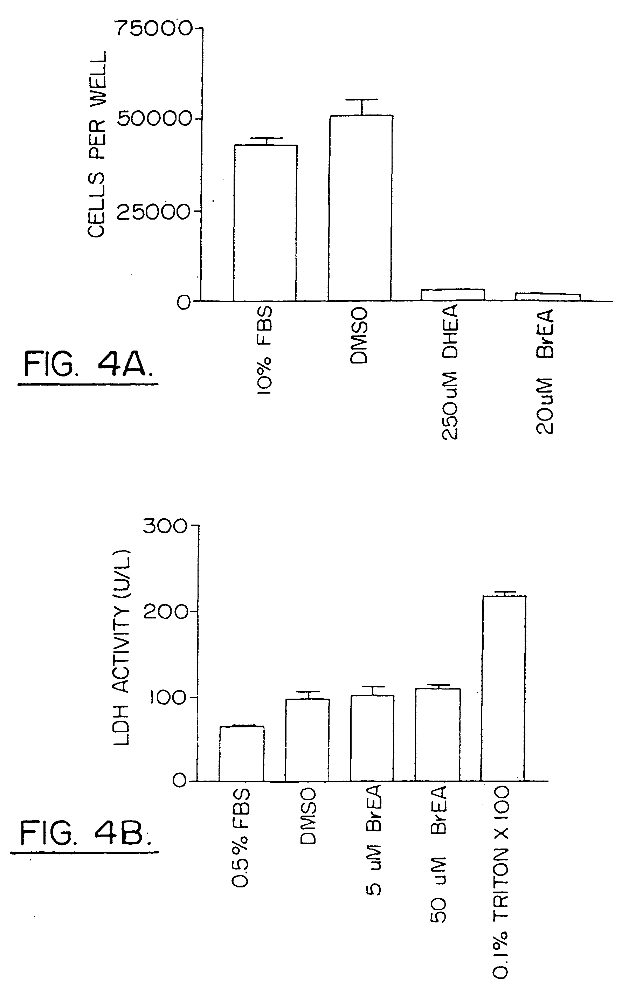

As shown in Figure 4A, growth inhibition cells by dehydroepiandrosterone (DHEA) and 16α-bromoepiandrosterone (BrEA) is confirmed by cell counts. Cells were cultured and cell numbers were quantitated as described above and in Figure 2. Each bar represented the mean direct visual count of stained cells in 6 experiments produced by 15,000 cells/well cultured for 24 hours.

Figure 4B shows that 16α-bromoepiandrosterone (BrEA) does not increase LDH activity in supernatant at 24 hours. In this example airway smooth muscle cells were grown to confluence and incubated with 5 µl/well DMSO vehicle or 16α-BrEA in vehicle for 24 hours before measurement of supernatant LDH activity, where 1.0 unit will reduce 1.0 µmole of pyruvate to L-lactate per minute at pH 7.5 at 37°C. Each bar represents the mean of 6 experiments. Similar results were seen after 2 hours incubation with inhibitor. There were no significant differences between cells treated with 16α-BrEA and those treated with DMSO vehicle alone.

DHEA and 16α-BrEA inhibit the activity of purified G6PDH from human erythrocytes and G6PDH activity when added directly to lysates of airway smooth muscle cells, as shown in Table I. However, when monolayers were pretreated with concentrations of DHEA or 16α-BrEA that inhibited cell proliferation, G6PDH activity of subsequent cell lysates was not significantly reduced (Table I).

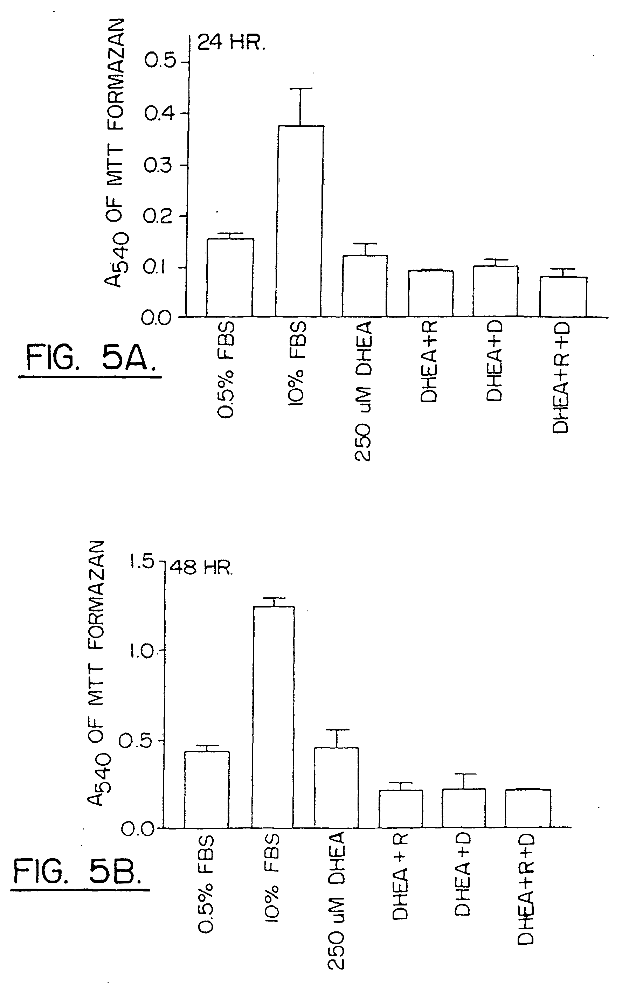

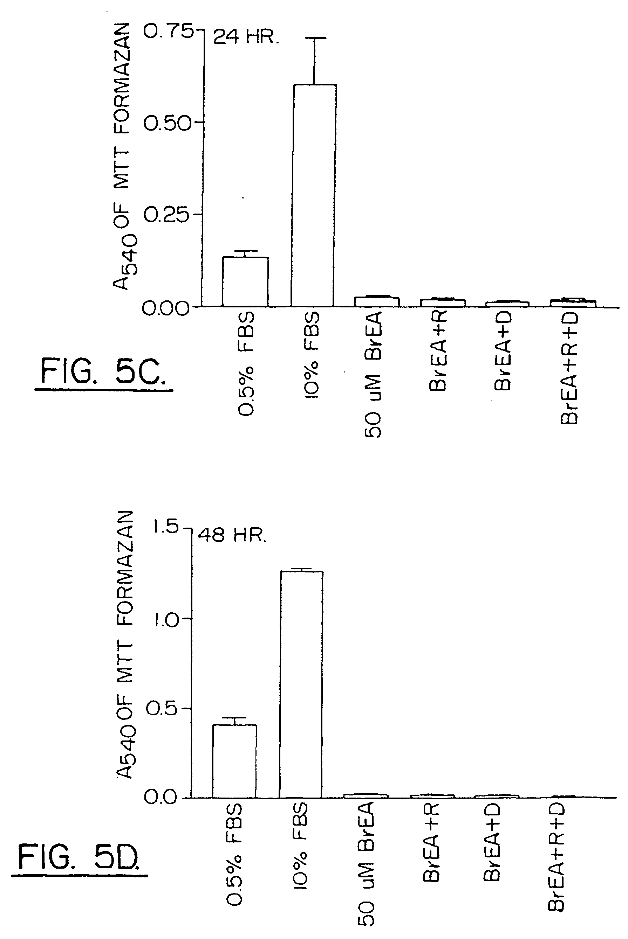

Also, in contrast to findings in malignant and immortalized cell lines, the addition of ribonucleosides and deoxyribonucleosides to culture medium failed to reverse growth inhibition from DHEA or 16α-BrEA (Figures 5A-D). Taken together, these data suggest that DHEA and 16α-BrEA do not impair proliferation of airway smooth muscle cells by disrupting hexose monophosphate shunt-dependent formation of ribose and deoxyribose sugars needed for RNA and DNA synthesis. In this example, cells were cultured and quantitated as before. Each bar represents mean MTT formazan absorbance in 4 experiments with 50,000 cells/well cultured for 24 hours (Figures 5A and 5C) or 48 hours (Figures 5B and 5D) in DMEM and 10% FBS in the presence or absence of DHEA or 16α-BrEA and 200 µM ribonucleosides (R, adenosine, guanosine, cytidine, and uridine) or deoxyribonucleosides (D, deoxyadenosine, deoxyguanosine, deoxycytidine, and thymidine).

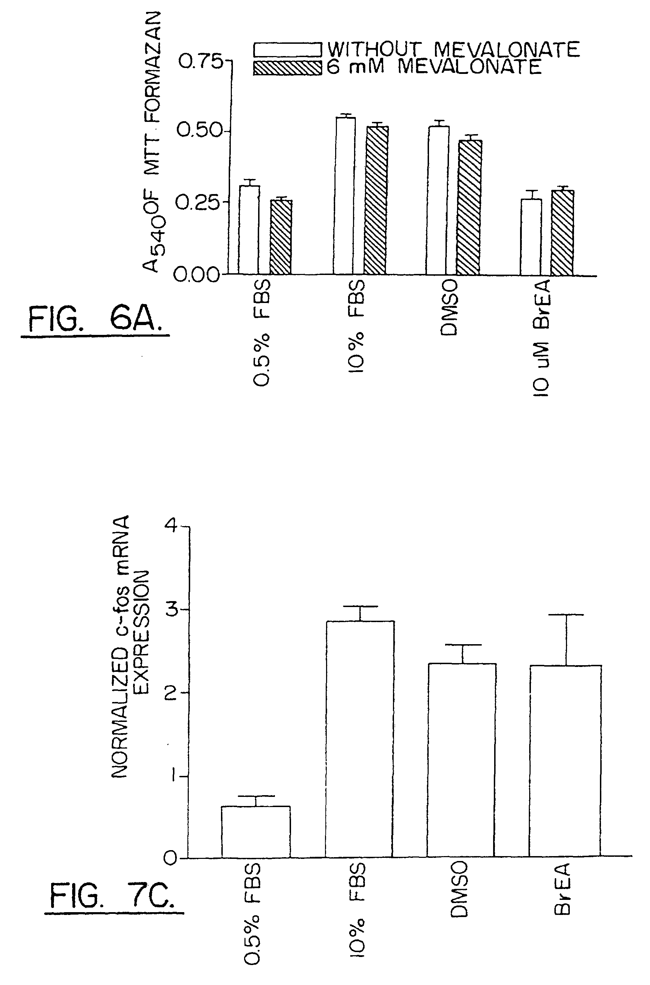

As shown in Figure 6A, supplementation of medium with mevalonic acid failed to overcome growth inhibition of airway smooth muscle monolayers by 16α-BrEa. In this example cells were cultured and quantitated as before. Each bar represents mean MTT formazan absorbance of 6 experiments of 50,000 cells/well cultured for 36 hours in with either 0.5% FBS, 10% FBS, 10% FBS + 5 µl DMSO vehicle, and 10% FBS + inhibitors in DMSO added to each well, in the presence or absence of 6 mM mevalonate in DMEM. Similar results were seen with DHEA.

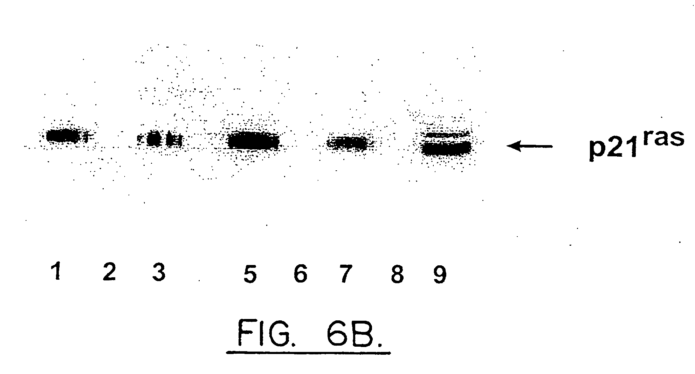

In Figure 6B, it is shown that treatment with 16α-BrEA does not deplete p21^{ras} in membranes of cultured airway smooth muscle cells. The gel shown is a representative immunoblot of 21 kd *Ras* protein in equal amounts of membrane protein fraction from confluent monolayers treated for 24 hours with 0.5% FBS (lane 1), 10% FBS (lane 3), 10% FBS + DMSO vehicle (lane 5) and 10% FBS + 10 µM 16α-BrEA (lane 7), respectively. Lanes 2, 4, 6 and 8 are immunoblots of corresponding supernatant fractions. Lane 9 represents an immunoblot of A431 cell lysate.

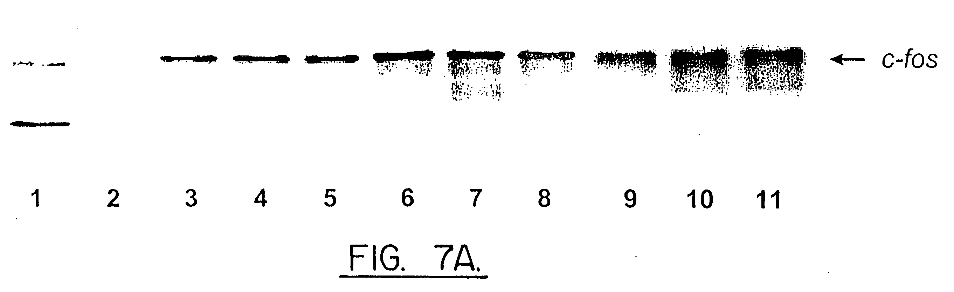

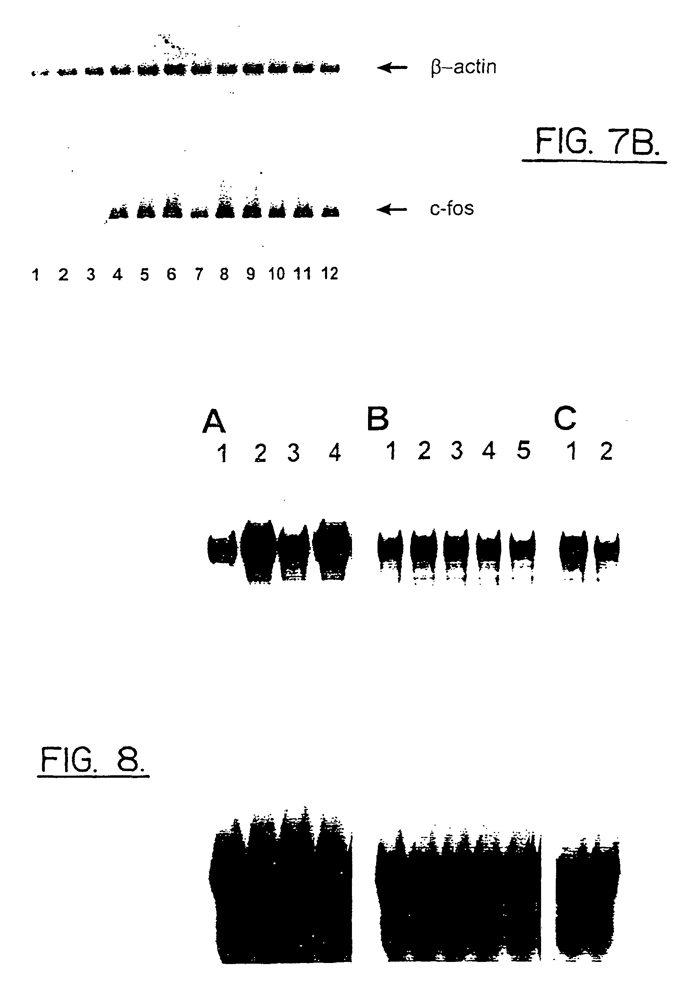

Finally, interference with *Ras*/*Raf*-mediated signal transduction would be expected to impair expression of early response genes such as *c-fos*. However, 16α-BrEA did not reduce stimulation of *c-fos* protein (Figure 7A) or mRNA (Figures 7B and 7C) seen normally in response to 10% FBS. These results suggest that DHEA and its analogs do not impair early signal transduction events important in airway smooth muscle for proliferative responses. Figure 7A is a representative immunoblot of *c-fos* protein from control monolayers treated with 0.5% FBS (lane 2) or 15 minutes (lanes 3-6), 30 minutes (lanes 6-8) and 60 minutes (lanes 9-11) after stimulation with 10% FBS. Cells in lanes 4, 7 and 10 were pretreated with DMSO vehicle 2 hr before stimulation. Cells in lanes 5, 8 and 11 were pretreated with 10 µM 16α-BrEA. Lane 1 is an immunoblot of A431 cell lysate. In Figure 7B, PCR gels of experiments were as follows: Lanes 1-3, 0.5% FBS control; lanes 4-6, 10% FBS control; lanes 7-9, 10% FBS pretreated with 10µM 16α-BrEA; lanes 10-12, 10% FBS pretreated with DMSO vehicle. In Figure 7C there is a summary of experiments shown in Figure 7B. Expression of *c-fos* mRNA is normalized to the housekeeping gene β-actin. In Figure 7C the expression of *c-fos* mRNA is normalized to the housekeeping gene β-actin.

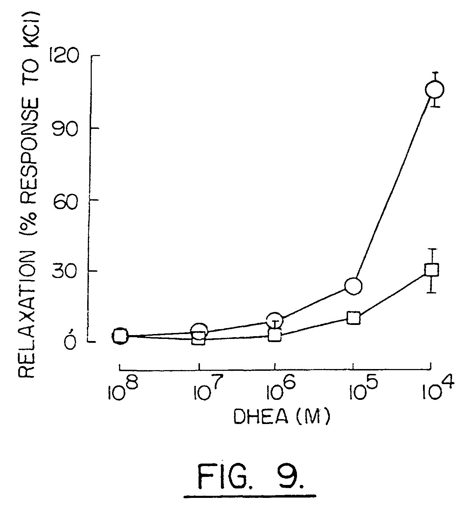

Figure 8 shows electrophoretic mobility shift assays of nuclear protein from airway smooth muscle cells pretreated with DHEA or 16α-BrEA for 2 hours prior to stimulation with 10% FBS. Nuclear protein was isolated after 6 hours and electrophoretic mobility shift assays (EMSAs) were performed. The Figures 8A-8B show representative gels from experiments performed at least 3 times with each inhibitor. In Figure 8A, EMSA of cells were pretreated with DHEA: Lane 1, 0.5% FBS; lane 2, 10% FBS, lane 3, 10% FBS + 50 µM DHEA; lane 4, 10% FBS + DMSO vehicle. In Figure 8B, EMSA of cells pretreated with 16α-BrEA: Lane 1, 0.5% FBS; lane 2, 10% FBS; lane 3, 10% FBS + DMSO vehicle; lane 4, 10% FBS + 2 µM 16α-BrEA; lane 5, 10% FBS + 10 µM 16α-BrEA. (Fig. 8C.). EMSA of cells stimulated with 10% FBS. The binding reaction in lane 2 was performed with an identical amount of nuclear protein as in lane 1, but in the presence of competition from 10X unlabeled wild-type oligonucleotide sequence for AP-1. The results show that DHEA (Figure 8A) and 16α-BrEA (Figure 8B) inhibit DNA binding of AP-1.
**Effect on bronchial smooth muscle contraction in vitro.** Figure 9 shows the concentration-effect curve to DHEA on isolated guinea pig main bronchi partially contracted with 3 x 10⁻² M KCl. Compared with its vehicle, it was found statistically different (P<0.01). The maximum concentration of DHEA caused 76.28±6.96% relaxation of the KCl-induced contraction (value obtained after subtraction of the reduction of force due to time-vehicle). The concentration of DHEA which produced 50% relaxation of the KCl-induced contraction was 2.16 x 10⁻⁵ M (GSEM=1.11). In Figure 9 open circles represent experiments with DHEA and open squares are with DMSO vehicle alone. Each point is mean ± SEM. n=6 for DHEA, n=5 for DMSO vehicle.

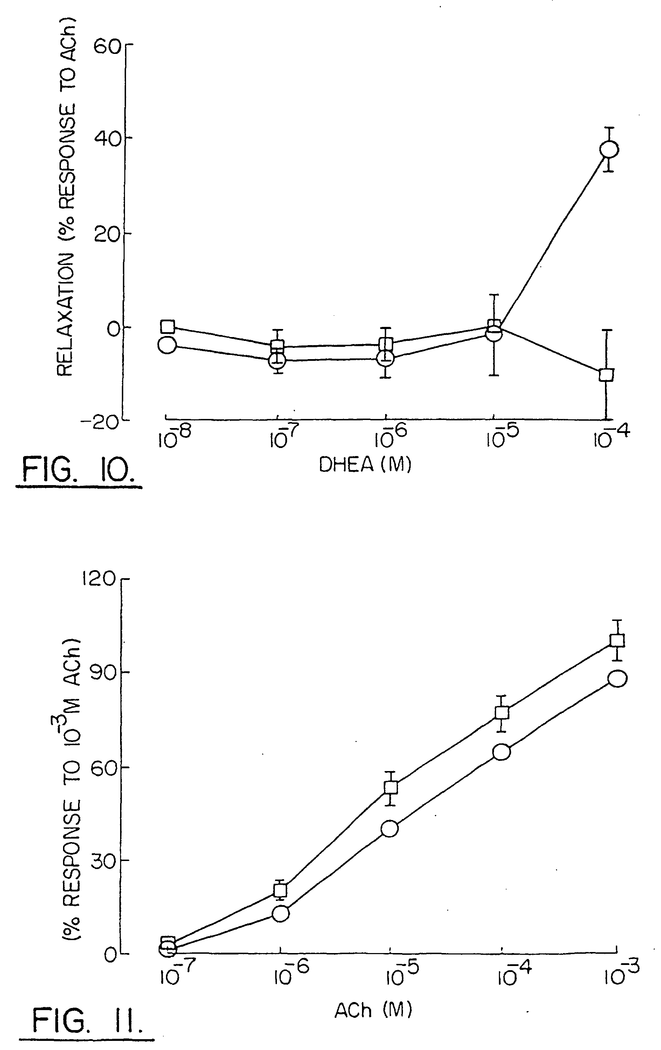

Figure 10 shows the effect of DHEA on bronchi partially contracted with 10⁻⁵ M ACh. 10⁻⁴ M DHEA caused 37.98±4.76% relaxation of the ACh-induced contraction (P<0.01 compared with its vehicle). In Figure 10 open circles represent experiments with DHEA and open squares are with vehicle alone. Each point is mean ± SEM and n=5.

The concentration-response curve to ACh was shifted to the right by the presence of 10⁻⁴ M DHEA (Figure 11). The ACh-EC₅₀ values were 1.4 x 10⁻⁵ M (geometric mean GSEM=1.2) and 8.4 x 10⁻⁶ M (geometric mean GSEM=1.3), respectively, in the presence and absence of 10⁻⁴ M DHEA (P<0.05). In Figure 11 open circles represent in the presence and open squares in the absence of 10⁻⁴ M DHEA. Each point is mean ± SEM and n=5.

Finally, 10⁻⁴ M DHEA reduced the rate of tension development in response to 10⁻⁵ ACh from 2.27±0.82 to 1.85±0.85 (P<0.05 by paired t test).
**Effect on interleukin-8 secretion by human airway epitheliumm in vitro.** As shown in Table II, 16α-BrEA caused a dose dependent inhibition of interleukin-8 (IL-8) secretion in response to stimulation of BEAS cells with either TNF, a potent pro-inflammatory cytokine present in many acute lung diseases, including asthma, and air pollution particulates. DMSO vehicle, as an antioxidant, reduces IL-8 secretion (a known effect) but BrEA inhibits it much more profoundly.

It has been shown that proliferation of rat airway smooth muscle cells is substantially reduced by treatment with pharmacologic concentrations of DHEA and 16α-bromoepiandrosterone (Figure 3). This inhibition was demonstrated both by metabolic assay related to cell number and a reduction in visual cell counts performed directly (Figure 4A).

In addition, DHEA and 16α-BrEA inhibit G6PD activity when added directly to a reaction mixture containing purified enzyme or cell lysate. (Table I). However, DHEA and 16α-BrEA did not inhibit G6PD activity when cell monolayers themselves were treated prior to enzyme harvest (Table I). Also, DHEA was more potent than 16α-BrEA as an inhibitor of G6PD acitvity in cell lysate, but the exact opposite was true for the two agents as inhibitors of airway smooth muscle proliferation (Figure 3). In addition, G6PD inhibition is thought to impair cell growth through interference with production of ribose and deoxyribose sugars needed for RNA and DNA synthesis, but exogenous provision of these factors by addition of ribo- and deoxyribonucleosides to growth media failed to overcome the inhibition of cell growth by DHEA and 16α-BrEA. Thus, the mechanism of inhibition of airway smooth muscle proliferation by DHEA and 16α-BrEA is unlikely to be interference with G6PD activity.

Addition of mevolonate to culture media did not overcome the growth inhibiting effects of 16α-BrEA (Figure 6A), nor did 16α-BrEA treatment of cells deplete *Ras* protein from cell membranes (Figure 6B). Also, treatment of monolayers with growth inhibiting concentrations of 16α-BrEA left normal expression of the early response gene *c-fos* intact (Figure 8), providing further evidence that DHEA and its analogs did not impair important *Ras*-mediated signal transduction events or disrupt function of the MAP-kinase pathway. These data suggest that DHEA and its analogs do not inhibit airway smooth muscle growth by interference with cholesterol biosynthesis, and secondarily, Ras-attachment to cell membranes and Ras-mediated signal transduction necessary for growth.

DHEA and 16α-BrEA decrease DNA binding of the transcription factor AP-1 in electrophoretic mobility shift assays performed with nuclear protein from treated cells (Figure 9). Transactivation of secondary response genes by activator protein-1 (AP-1) is an important point of convergence of multiple pathways through which many growth factors stimulate cell proliferation (Angel, P, and Karin, M. "The role of Jun, Fos and the AP-1 complex in cell-proliferation and transformation," *Biochim*. *Biophys*. *Acta* 1072:129-157, 1991). Transrepression of AP-1 has been shown to account for the antiproliferative effects of glucocorticoids (Heck, S., Kullmann, M., Gast, Al, Ponta, H., Rahmsdorf, H.J., Herrlich, Pl., and Cata, A.C.B., "A distinct modulating domain in glucocorticoid receptor monomers in the repression of activity of the transcription factor AP-1," *EMBO J*. 17:4087-4095, 1994). Although DHEA is not a glucocorticoid, it too has been reported to interact with cytoplasmic steroid receptors (Meikel, A.W., Dorchuck, R.W., Araneo, B.A, Stringham, J.D., Evans, T.G, Spruance, S.L., and Daynes, R.A., "The presence of a dehydroepiandrosterone-specific receptor binding complex in murine T cells", *J*. *Steroid Biochem*. *Molec*. *Biol*. 42:293-304, 1992). Thus, it is likely that DHEA and its analogs also interfere with DNA binding of AP-1, thereby interrupting AP-1 mediated signal transduction processes and inhibiting proliferative responses to a wide variety of mitogens, including PDGF and those found in fetal bovine serum. Interruption of AP-1 responses, therefore, provide the best explanation of how DHEA and analogs impair smooth muscle proliferation.

In addition to preventing airway smooth muscle proliferation and remodeling, DHEA and it analogs could have other potential therapeutic benefits in asthma.

First, DHEA and its analogs might be useful as glucocorticoid sparing agents. Acquired glucocorticoid resistance has been hypothesized to occur in part by cytokine-induced overexpression of AP-1 complexes that bind and transrepress the activated glucocorticoid receptor complex (Adcock, Lane, *et al*. (1995), *supra*). The DHEA or analog/receptor complex could instead sacrificially bind AP-1, freeing activated glucocorticoid receptors for attachment to GREs, thus overcoming the relative glucocorticoid resistance of the inflammatory state.

Second, DHEA and its analogs may have anti-inflammatory activity in their own right. Many immunoregulatory genes contain AP-1 promoter sites, and inhibition of AP-1 could negatively impact on expression of pro-inflammatory cytokines. Also, DHEA has been reported to reduce activation of the transcription factor nuclear factor κB (NF-κB) (Yang, Y.U., Schwartz, A., and Henderson, E.E., "Inhibition of HIV-1 latency reactivation by dehydroepiandrosterone (DHEA) and an analog of DHEA," *AIDS Res*. *Hum*. *Retroviruses*, 9, 747-754 (1993). It was also found that 16α-BrEA reduces tumor necrosis factor- and particulate air pollution-stimulated secretion of interleukin-8 by cultured human respiratory epithelium (Table II). Reduction of the secretion of proinflammatory cytokines would be expected to reduce overall inflammatory responses within the asthmatic airway.

Third, functioning as a Ca²⁺-activated K⁺ channel (K_{Ca}) opener (Peng, W., Hoidal, J.R., and Farrukh, I.S., "Dehydroepiandrosterone, a novel Ca²⁺-activated K⁺ channel opener," *Am*. *Rev*. *Respir*. *Crit*. *Care Med*., 155, A790 (1997, abstract), DHEA can relax systemic (Barbagallo, M., Shan, J., Pang, P.K.T., and Resnick, L.M., "Effects of dehydroepiandrosterone sulfate on cellular calcium responsiveness and vascular contractility," *Hypertension*, 26, 1065-1069 (1995) and pulmonary arterial (Farrukh, I.S., Peng, W., Orlinska, U., and Hoidal, J.R., "Dehydrcepiandrosterone -mediated pulmonary vasodilation of hypoxic ferret lungs: a novel mechanism," *Am*. *J. Respir. Crit*. *Care Med*., 153, A586 (1996, abstract) vessels contracted by a variety of agonists, and reverses KCl-induced contraction of guinea pig tracheal smooth muscle. Thus, DHEA has direct bronchodilator activity in airway smooth muscle. Also, DHEA and analogs may be synergistic with beta-adrenergic bronchodilators in relaxing airway smooth muscle. K_{Ca} channels are implicated as important target proteins for beta-2-adrenoceptor agonist-induced relaxation (Kume, H., Takail, A., Tokuno, H., and Tomita, T., "Regulation of Ca²⁺-dependent K⁺-channel activity in tracheal myocytes by phosphorylation", *Nature*, 341, 152 (1989); Jones, T.R., Charette, L, Garcia, M.L., and Kaczorowski, G.J., "Selective inhibition of relaxation of guinea-pig trachea by charybdotoxin, a potent Ca⁺⁺-activated K⁺ channel inhibitor", *J*. *Pharmacol*. *Exp*. *Ther*., 255, 697 (1990); Miura, M., Belvisi, M.G., Stretton, C.D., Yacoub, M.H., and Barnes, P.J., "Role of potassium channels in bronchodilator responses in human airways", *Am*. *Rev*. *Respir*. *Dis*. 146:132, 1992; Kume, H., Grazizno, M.P., and Kotlikoff, M.I., "Stimulatory and inhibitory regulation of calcium-activated potassium channels by guanine nucleotide-binding proteins", *Proc*. *Natl*. *Acad*. *Sci*., *U*.*S*.*A*., 89, 110551 (1992). These channels are densely distributed in the cell membranes of airway smooth muscle in humans (Snetkov, V.A, Hirst, S.J., Twort, C.H.C., and Ward,. J.P.T., "Potassium currents in human freshly isolated bronchial smooth muscle cells", *Br*. *J*. *Pharmacol*., 115, 1117 (1995).

Beta-agonists open K_{Ca} channels either by increasing intracellular cAMP, thereby activating protein kinase A, which in turn phosphorylates the K_{Ca} (Kume, H., Takail, A., Tokuno, H., and Tomita, T., "Regulation of Ca²⁺-dependent K⁺-channel activity in tracheal myocytes by phosphorylation", *Nature*, 341, 152 (1989), or beta-agonists stimulate K_{Ca} activity by a G protein-dependent, protein kinase A-independent mechanism (Yatani, A., Codina, J., Imoto, Y., Reeves, J.P., Birnbaumer, L, and Brown, A.M., "A G protein directly regulates mammlian cardiac calcium channels", *Science,* 238, 1288 (1987); Yatanni, A., Imoto, Y., Codina, J., Hamilton, S.L., Brown, A.M., and Birnbaumer, L., "The stimulatory G protein of adenylylcyclose, Gₛ also sstimulates dihydropyridine-sensitive Ca²⁺ channels. Evidence of direct regulation independent of phosphorylation by cAMP-dependent protein kinase or stimulation by a dihydropyridine agonist", *J*. *Biol*. *Chem*., 263, 9987 (1988). Both of these beta-agonist dependent mechanisms are susceptible to inhibition by phenonemonen such as beta-receptor desensitization, a common problem in the inflammed airways of asthmatics. DHEA would offer a "bypass" approach toward activating K_{Ca} channels directly, without having to go through beta-agonist-receptor interactions. This might be especially useful in the acute asthmatic, who is already relatively insensitive to bronchial relaxation by beta agonists.

Vagally induced airways hyperreactivity in asthma is caused by failure of function of the neuronal M₂ muscarinic receptor on the vagus nerves. These receptors normally function to inhibit acetylcholine release from the vagus, thus limiting vagally induced bronchoconsstriction. Neuronal M₂ muscarinic receptors are not functioning in asthma (Ayala, L.E., and Ahmed, T., "Is there loss of a protective muscarinic receptor in asthma?", *Chest*, 96,1285-1291 (1989); Minette P., and Barnes, P.J., "Prejunctional inhibitory muscarinic receptors in cholinergic nerve terminals in human and guinea-pig airways", *J*. *Appl*. *Physiol*., 64, 2532-2537 (1988), causing any irritant stimulating vagal reflexes to produce exaggerated bronchoconstriction in the asthma patient. By inhibiting acetylcholine-induced bronchoconstriction, DHEA and its analogs could reduce generalized vagally-mediated airways hyperreactivity in asthma that contributes overall to asthmatic symptoms.

Finally, DHEA treatment in vitro enhances T-lymphocyte production of interleukin-2 (Meikle, et al. (1992), supra, raising the possibility that DHEA or analogs might promote reversion of asthmatic airway lymphocytes from TH2 back to TH1 state. See Robinson, D.S., Hamid, Q., Ying, S., Tsicopoulos, A., Barkans, J., Bentley, A.M., Corrigan, C., Durham, S.R., and Kay, A.B., "Predominant TH2-like bronchoalveolar T-lymphocyte population in atopic asthma," *N*. *Engl*. *J*. *Med*., 326, 298-304 (1992).

It has been demonstrated that based on the antiproliferative effects, DHEA and its analogs offer utility as a treatment for airway smooth muscle remodeling in man. An advantage of these agents over glucocorticoids is their slightly anabolic and anti-glucocorticoid spectrum of effects. See Regelson, W., and Kalimi, M., "Dehydroepiandrosterone (DHEA)--the multifunctional steroid. II. Effects on the CNS, cell proliferation, metabolic and vascular, clinical and other effects. Mechanism of action?" Ann. N.Y. Acad. Sci., 719, 564-575 (1994). Thus, DHEA or its analogs might even provide a new approach to the treatment of airways diseases without the adverse long term consequences of corticosteroid use on blood pressure or glucose and bone metabolism.

## Claims

1. Use of a compound having the following formula: 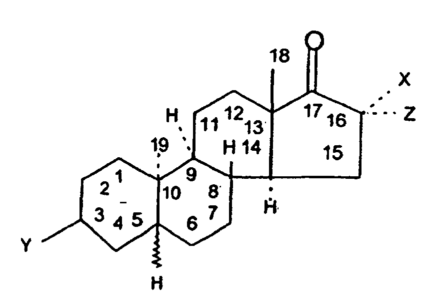 OR 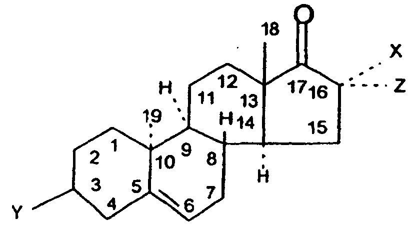 wherein
X is halogen, hydroxy or hydrogen;
Y is hydrogen;
Z is hydrogen
in the preparation of a medicament comprising a pharmaceutically effective amount of said compound for treating chronic asthma in an animal suffering therefrom.

2. Use according to Claim 1, wherein said compound is selected from a member of the group consisting of 16α-bromo-5-androsten-17-one, 16β-bromo-5-androsten-17-one, 16α-fluoro-5-androsten-17-one, 16β-fluoro-5-androsten-17-one, 16α-bromo-5-androstan-17-one, 16β-bromo-5-androstan-17-one, 16α-fluoro-5-androstan-17-one, and 16β-fluoro-5-androstan-17-one.

3. Use according to Claim 1, wherein an effective amount of compound is from 2 to 10 mg/kg/day.

4. Use according to Claim 1, wherein said compound is 16-bromo or fluorinated analog and said effective amount of compound is from 0.2 to 2 mg/kg/day.

5. Use according to Claim 1, wherein said medicament is formulated for administration by aerosilization.

6. Use according to Claim 1, wherein said medicament is formulated for administration by metered dose inhaler.

7. Use according to Claim 1, wherein said medicament is formulated for administration by powder dose inhaler.

8. Use according to Claim 1, wherein said medicament is formulated for administration by inhalation of drug formulated as part of a liposome carrier.

9. Use according to Claim 1, wherein said medicament is formulated for administration of said compound directly into the respiratory tract.

10. Use according to Claim 1, wherein said medicament is for inhibiting bronchoconstriction.

11. Use according to Claim 1, wherein said medicament is for inhibiting asthma related secretion of inflammatory cytokines by airway epithelium.

12. Use according to Claim 1, wherein said medicament is for inhibiting acetylcholine mediated, vagal airways hyperactivity in asthma.

13. Use according to Claim 1, wherein said medicament is for potentiating bronchodilator activity of beta agonist bronchodilators.

14. Use according to Claim 1 wherein the compound is 16α-fluoro-5-androsten-17-one.

15. Use according to Claim 1 wherein the compound is 16α-fluoro-5-androstan-17-one.

16. Use of a compound of the formula: 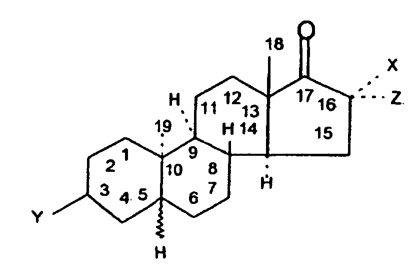 OR 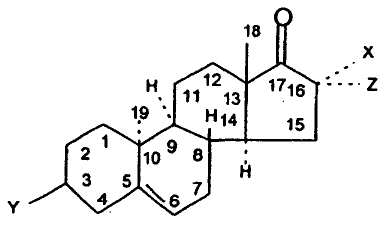 wherein
X is halogen, hydroxy or hydrogen;
Y is hydrogen;
Z is hydrogen,
in the preparation of a medicament comprising a pharmaceutically effective amount of said compound for inhibiting airway smooth muscle proliferation in the treatment of asthma in an animal.

17. Use of a compound of the formula: 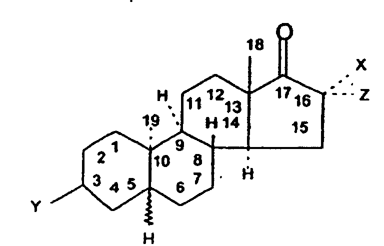 OR 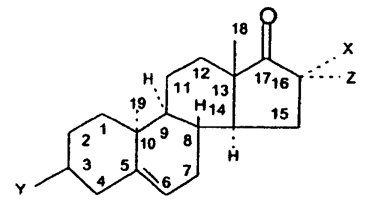 wherein
X is halogen, hydroxy or hydrogen;
Y is hydrogen;
Z is hydrogen,
in the preparation of a medicament comprising a pharmaceutically effective amount of a compound for inhibiting DNA binding of AP-1 in the treatment of asthma in an animal.

18. Use according to Claim 17, wherein said medicament is for reduction of glucocorticoid insensitivity in asthma by inhibition of AP-1.

19. Use according to Claim 16 or 17 wherein said compound is selected from a member of the group consisting of 16α-bromo-5-androsten-17-one, 16β-bromo-5-androsten-17-one, 16α- fluoro -5-androsten-17-one, 16β-fluoro-5-androsten-17-one, 16α-bromo-5-androstan-17-one, 16β-bromo-5-androstan-17-one, 16α-fluoro-5-androstan-17-one, and 16β-fluoro-5-androstan-17-one.

20. Use according to Claim 19, wherein said compound is 16α-fluoro-5-androsten-17-one or 16α-fluoro-5-androstan-17-one.

21. Use according to any one of claims 16-20 wherein said medicament is formulated for administration by aerosolization.

22. Use according to any one of claims 16-20 wherein said medicament is formulated for administration by metered dose inhaler.

23. Use according to any one of claims 16-20 wherein said medicament is formulated for administration by powder dose inhaler.

24. Use according to any one of claims 16-20 wherein said medicament is formulated for administration by inhalation of drug formulated as part of a liposome carrier.

25. Use according to any one of claims 16-20 wherein said medicament is formulated for administration of said compound directly into the respiratory tract.

26. Use according to any one of Claims 1-25 wherein said animal is man.

## Patentansprüche

1. Verwendung einer Verbindung mit der folgenden Formel: 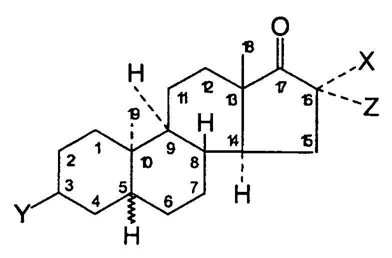 oder 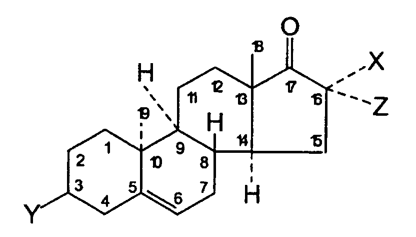 worin
X Halogen, Hydroxy oder Wasserstoff ist;
Y Wasserstoff ist;
Z Wasserstoff ist;
in der Herstellung eines Medikaments, das eine pharmazeutisch wirksame Menge der Verbindung zur Behandlung von chronischem Asthma eines daran leidenden Tieres umfasst.

2. Verwendung gemäss Anspruch 1, worin die Verbindung aus einem Vertreter der Gruppe ausgewählt ist, die aus 16α-Brom-5-androsten-17-on, 16β-Brom-5-androsten-17-on, 16α-Fluor-5-androsten-17-on, 16β-Fluor-5-androsten-17-on, 16α-Brom-5-androstan-17-on, 16β-Brom-5-androstan-17-on, 16α-Fluor-5-androstan-17-on und 16β-Fluor-5-andostran-17-on besteht.

3. Verwendung gemäss Anspruch 1, worin eine wirksame Menge der Verbindung 2 bis 10 mg/kg/Tag beträgt.

4. Verwendung gemäss Anspruch 1, worin die Verbindung 16-Brom oder ein fluoriertes Analogon ist und die wirksame Menge der Verbindung 0,2 bis 2 mg/kg/Tag beträgt.

5. Verwendung gemäss Anspruch 1, worin das Medikament zur Verabreichung durch Aerosolisierung formuliert ist.

6. Verwendung gemäss Anspruch 1, worin das Medikament zur Verabreichung durch einen Dosierinhalator formuliert ist.

7. Verwendung gemäss Anspruch 1, worin das Medikament zur Verabreichung durch einen Pulverdosisinhalator formuliert ist.

8. Verwendung gemäss Anspruch 1, worin das Medikament zur Verabreichung durch Inhalation von Wirkstoff formuliert ist, der als Teil eines Liposomenträgers formuliert ist.

9. Verwendung gemäss Anspruch 1, worin das Medikament zur Verabreichung der Verbindung direkt in die Atemwege formuliert ist.

10. Verwendung gemäss Anspruch 1, worin das Medikament zur Unterdrückung von Bronchokonstriktion ist.

11. Verwendung gemäss Anspruch 1, worin das Medikament zur Unterdrückung von Asthma-bezogener Sekretion von inflammatorischen Cytokinen durch das Atemwegsepithel ist.

12. Verwendung gemäss Anspruch 1, worin das Medikament zur Unterdrückung von Acetylcholin-vermittelter vagaler Atemwegshyperaktivität bei Asthma ist.

13. Verwendung gemäss Anspruch 1, worin das Medikament zur Potenzierung der Bronchodilatatoraktivität von β-Agonist-Bronchodilatatoren ist.

14. Verwendung gemäss Anspruch 1, worin die Verbindung 16α-Fluor-5-androsten-17-on ist.

15. Verwendung gemäss Anspruch 1, worin die Verbindung 16α-Fluor-5-androstan-17-on ist.

16. Verwendung einer Verbindung der Formel: 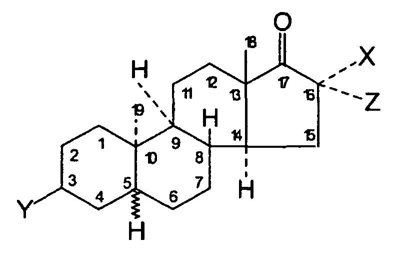 oder 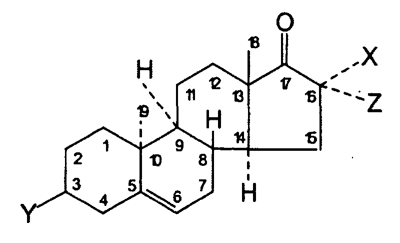 worin
X Halogen, Hydroxy oder Wasserstoff ist;
Y Wasserstoff ist;
Z Wasserstoff ist;
in der Herstellung eines Medikaments, das eine pharmazeutisch wirksame Menge der Verbindung zur Inhibierung der Proliferation der glatten Atemwegsmuskulatur in der Behandlung von Asthma eines Tieres umfasst.

17. Verwendung einer Verbindung der Formel: 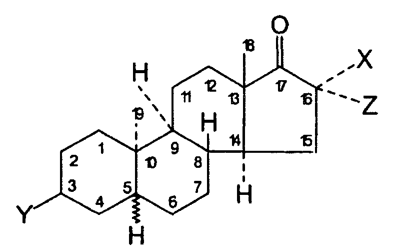 oder 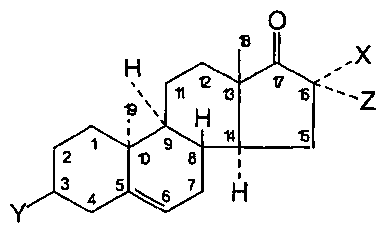 worin
X Halogen, Hydroxy oder Wasserstoff ist;
Y Wasserstoff ist;
Z Wasserstoff ist;
in der Herstellung eines Medikaments, das eine pharmazeutisch wirksame Menge der Verbindung zur Inhibierung der DNA-Bindung von AP-1 in der Behandlung von Asthma eines Tieres umfasst.

18. Verwendung gemäss Anspruch 17, worin das Medikament zur Reduktion der Glukokortikoidunempfindlichkeit bei Asthma durch Inhibierung von AP-1 ist.

19. Verwendung gemäss Anspruch 16 oder 17, worin die Verbindung aus einem Vertreter der Gruppe ausgewählt ist, die aus 16α-Brom-5-androsten-17-on, 16β-Brom-5-androsten-17-on, 16α-Fluor-5-androsten-17-on, 16β-Fluor-5-androsten-17-on, 16α-Brom-5-androstan-17-on, 16β-Brom-5-androstan-17-on, 16α-Fluor-5-androstan-17-on und 16β-Fluor-5-andostran-17-on besteht.

20. Verwendung gemäss Anspruch 19, worin die Verbindung 16α-Fluor-5-androsten-17-on oder 16α-Fluor-5-androstan-17-on ist.

21. Verwendung gemäss einem der Ansprüche 16 bis 20, worin das Medikament zur Verabreichung durch Aerosolisierung formuliert ist.

22. Verwendung gemäss einem der Ansprüche 16 bis 20, worin das Medikament zur Verabreichung durch einen Dosierinhalator formuliert ist.

23. Verwendung gemäss einem der Ansprüche 16 bis 20, worin das Medikament zur Verabreichung durch einen Pulverdosisinhalator formuliert ist.

24. Verwendung gemäss einem der Ansprüche 16 bis 20, worin das Medikament zur Verabreichung durch Inhalation von Wirkstoff formuliert ist, der als Teil eines Liposomenträgers formuliert ist.

25. Verwendung gemäss einem der Ansprüche 16 bis 20, worin das Medikament zur Verabreichung der Verbindung direkt in die Atemwege formuliert ist.

26. Verwendung gemäss einem der Ansprüche 1 bis 25, worin das Tier der Mensch ist.

## Revendications

1. Utilisation d'un composé de formule 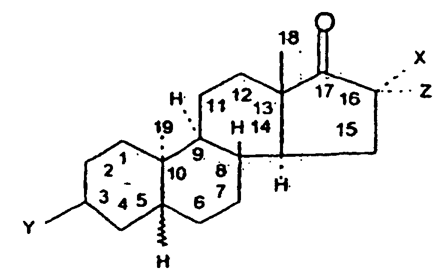 ou 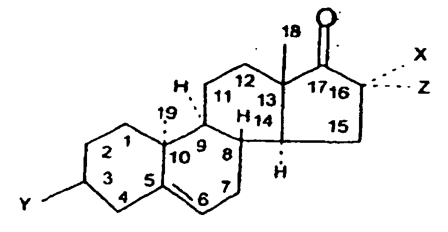 dans laquelle
X représente un atome d'halogène, un groupe hydroxy ou un atome d'hydrogène,
Y représente un atome d'hydrogène,
Z représente un atome d'hydrogène,
pour la préparation d'un médicament comprenant une quantité pharmaceutiquement efficace dudit composé, destiné à traiter l'asthme chronique chez un animal souffrant d'asthme chronique.

2. Utilisation selon la revendication 1, dans laquelle ledit composé est choisi parmi les éléments du groupe formé par la 16α-bromo-5-androstén-17-one, la 16β-bromo-5-androstén-17-one, la 16α-fluoro-5-androstén-17-one, la 16β-fluoro-5-androstén-17-one, la 16α-bromo-5-androstan-17-one, la 16β-bromo-5-androstan-17-one, la 16α-fluoro-5-androstan-17-one et la 16β-fluoro-5-androstan-17-one.

3. Utilisation selon la revendication 1, dans laquelle la quantité efficace d'un composé est comprise entre 2 et 10 mg/kg/jour.

4. Utilisation selon la revendication 1, dans laquelle ledit composé est un dérivé 16-bromo ou un analogue fluoré et ladite quantité efficace est comprise entre 0,2 et 2 mg/kg/jour.

5. Utilisation selon la revendication 1, dans laquelle ledit médicament est formulé pour être administré sous forme d'aérosol.

6. Utilisation selon la revendication 1, dans laquelle ledit médicament est formulé pour être administré au moyen d'un aérosol-doseur.

7. Utilisation selon la revendication 1, dans laquelle ledit médicament est formulé pour être administré au moyen d'un aérosol-doseur de poudre.

8. Utilisation selon la revendication 1, dans lequel ledit médicament est formulé pour être administré par inhalation d'un principe actif incoporé dans un véhicule liposomique.

9. Utilisation selon la revendication 1, dans laquelle ledit médicament est formulé pour permettre l'administration du composé directement dans les voies respiratoires.

10. Utilisation selon la revendication 1, dans laquelle ledit médicament est destiné à inhiber la bronchoconstriction.

11. Utilisation selon la revendication 1, dans laquelle ledit médicament est destiné à inhiber la sécrétion, liée à l'asthme, de cytokines inflammatoires par l'épithélium des voies respiratoires.

12. Utilisation selon la revendication 1, dans laquelle ledit médicament est destiné à inhiber l'hyperactivité vagale des voies respiratoires, médiée par l'acétylcholine, de sujets asthmatiques.

13. Utilisation selon la revendication 1, dans laquelle ledit médicament est destiné à potentialiser l'activité brochodilatatrice de bronchodilatateurs du groupe des bêta-agonistes.

14. Utilisation selon la revendication 1, dans laquelle le composé est la 16α-fluoro-5-androstén-17-one.

15. Utilisation selon la revendication 1, dans laquelle le composé est la 16α-fluoro-5-androstan-17-one.

16. Utilisation d'un composé de formule 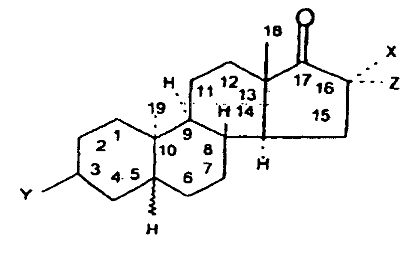 ou 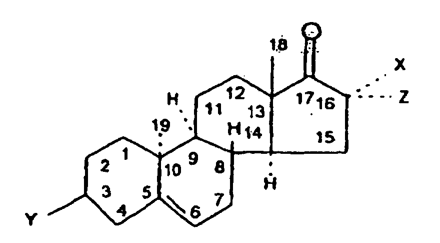 dans laquelle
X représente un atome d'halogène, un groupe hydroxy ou un atome d'hydrogène,
Y représente un atome d'hydrogène,
Z représente un atome d'hydrogène,
pour la préparation d'un médicament comprenant une quantité pharmaceutiquement efficace dudit composé, destiné à inhiber la prolifération de muscles lisses des voies respiratoires dans le cadre d'un traitement de l'asthme chez un animal.

17. Utilisation d'un composé de formule 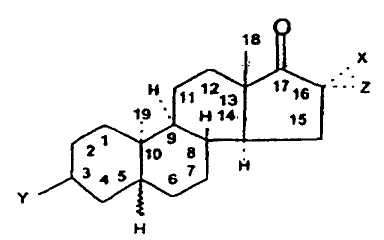 ou 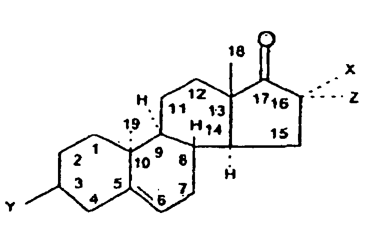 dans laquelle
X représente un atome d'halogène, un groupe hydroxy ou un atome d'hydrogène,
Y représente un atome d'hydrogène,
Z représente un atome d'hydrogène,
pour la préparation d'un médicament comprenant une quantité pharmaceutiquement efficace d'un composé destiné à inhiber la liaison de l'AP-1 à l'ADN dans le traitement de l'asthme chez un animal.

18. Utilisation selon la revendication 17, dans laquelle ledit médicament est destiné à la réduction de l'insensibilité aux glucocorticoïdes de sujets asthmatiques par inhibition de l'AP-1.

19. Utilisation selon la revendication 16 ou 17, dans laquelle ledit composé est choisi parmi les éléments du groupe formé par la 16α-bromo-5-androstén-17-one, la 16β-bromo-5-androstén-17-one, la 16α-fluoro-5-androstén-17-one, la 16β-fluoro-5-androstén-17-one, la 16α-bromo-5-androstan-17-one, la 16β-bromo-5-androstan-17-one, la 16α-fluoro-5-androstan-17-one et la 16β-fluoro-5-androstan-17-one.

20. Utilisation selon la revendication 19, dans laquelle ledit composé est la 16α-fluoro-5-androstén-17-one ou la 16α-fluoro-5-androstan- 17-one.

21. Utilisation selon l'une quelconque des revendications 16 - 20, dans laquelle ledit médicament est formulé pour être administré sous forme d'aérosol.

22. Utilisation selon l'une quelconque des revendications 16 - 20, dans laquelle ledit médicament est formulé pour être administré au moyen d'un aérosol-doseur.

23. Utilisation selon l'une quelconque des revendications 16 - 20, dans laquelle ledit médicament est formulé pour être administré au moyen d'un aérosol-doseur de poudre.

24. Utilisation selon l'une quelconque des revendications 16 - 20, dans lequel ledit médicament est formulé pour être administré par inhalation d'un principe actif incorporé dans un véhicule liposomique.

25. Utilisation selon l'une quelconque des revendications 16 - 20, dans laquelle ledit médicament est formulé pour permettre l'administration du composé directement dans les voies respiratoires.

26. Utilisation selon l'une quelconque des revendications 1 à 25 dans laquelle ledit animal est l'homme.
